# EUROPÄISCHE PATENTSCHRIFT

(11) **EP 4 061 294 B1**
(45) Veröffentlichungstag und Bekanntmachung des Hinweises auf die Patenterteilung: **22.01.2025**
(21) Anmeldenummer: 20808330.3
(22) Anmeldetag: 16.11.2020
(51) Int. Cl.: A61F 5/01, F16F 9/32

(54) **VORRICHTUNG ZUR STABILISIERUNG VON BEWEGUNGEN ZWEIER SICH RELATIV ZUEINANDER BEWEGBARER TEILE EINES MENSCHLICHEN KÖRPERBEREICHS UND/ODER EINES SPORTGERÄTS MIT SCHWENKBAREM KRAFTÜBERTRAGUNGSELEMENT**
DEVICE FOR STABILISING MOVEMENTS OF TWO PARTS OF A HUMAN BODY REGION AND/OR OF A SPORTS APPLIANCE WHICH ARE MOVABLE RELATIVE TO EACH OTHER, HAVING A PIVOTABLE FORCE TRANSMISSION ELEMENT
DISPOSITIF DE STABILISATION DE MOUVEMENTS DE DEUX PARTIES D'UNE RÉGION DE CORPS HUMAIN ET/OU D'UN APPAREIL DE SPORTS QUI SONT MOBILES L'UNE PAR RAPPORT À L'AUTRE, AYANT UN ÉLÉMENT DE TRANSMISSION DE FORCE PIVOTANT

(30) Priorität: 20.11.2019 DE 102019131377
(43) Veröffentlichungstag der Anmeldung: 28.09.2022
(73) Patentinhaber: Betterguards Technology GmbH, 10625 Berlin (DE)
(72) Erfinder: BICHLER, Vinzenz, 10777 Berlin (DE)
(74) Vertreter: Okoampah, Rene
(86) Internationale Anmeldenummer: PCT/EP2020/082301
(87) Internationale Veröffentlichungsnummer: WO 2021/099276

(56) Entgegenhaltungen:
- EP-A1- 2 984 956
- EP-A1- 3 092 980
- WO-A1-2018/020020
- DE-A1- 102017 112 911
- DE-A1- 102017 117 786
- DE-B3- 102018 116 569
- US-A- 5 103 811
- US-A1- 2013 081 304

## Beschreibung

### Technisches Gebiet

Die vorliegende Erfindung betrifft eine Schuhsohle umfassend eine Vorrichtung zur Stabilisierung von Bewegungen zweier sich relativ zueinander bewegbarer Teile der Schuhsohle gemäß Anspruch 1. Weitere vorteilhafte Ausgestaltungen sind in den abhängigen Ansprüchen offenbart.

### Stand der Technik

Es ist bekannt Körpergelenke, Muskeln und Sehnen mittels Vorrichtungen, welche eine geschwindigkeitsabhängige Bewegungsbegrenzung ermöglichen, zu stabilisieren. Beispielsweise kann die Bewegung des Sprunggelenks mittels orthopädischer Schuhe oder Vorrichtungen geschwindigkeitsabhängig stabilisiert werden, um Traumata infolge eines Umknickens, mithin einer Bewegung des Sprunggelenks über zumindest eines seiner Sprunggelenksachsen in einem unphysiologischen, das heißt für das entsprechend zu stabilisierende Körperteil kritischen, Bereich zu verhindern. Unter anderem wird das geschwindigkeitsabhängige Verhalten solcher Vorrichtungen dadurch erreicht, dass sich zwei Körper relativ zueinander bewegen, wobei sich zwischen den Körpern ein Fluid befindet, welches in einer Kammer von relativ zueinander bewegbaren Komponenten verdrängt wird.

Derartige Vorrichtungen können zwischen zwei Körperstellen eines Anwenders fixiert werden. Dabei bildet ein Teil einer solchen Vorrichtung eine Aufnahme, welche mit dem Fluid gefüllt ist. Der andere Teil bildet einen Auszugskörper, welcher in der Aufnahme ein- und ausziehbar angeordnet ist. Werden über die beiden Körperstellen des Anwenders unphysiologische Kräfte in die Vorrichtung eingeleitet, resultiert dies in einer Ein- oder Ausziehbewegung des Auszugskörpers. Dies wiederum ruft in der Aufnahme einen erhöhten Widerstand des Füllmediums hervor, wodurch eine Relativbewegung zwischen dem Auszugskörper und der Aufnahme nur noch unter sehr hohem Kraftaufwand möglich ist. In manchen Vorrichtungen wird dieser Effekt noch dadurch erhöht, dass das Füllmedium geschwindigkeits- bzw. impulsabhängige Materialeigenschaften aufweist, beispielsweise scherverdickende Eigenschaften. Mit derartigen Vorrichtungen am Körper oder an einem Bekleidungsgegenstand kann die Stützwirkung maximiert werden, wenn deren Ein- und Auszugsrichtung entlang zweier Punkte verläuft, die bei der betreffenden Bewegung die größte Abstandsänderung erfahren.

Trotz der hervorragenden Praktikabilität derartiger Vorrichtungen hat sich aber gezeigt, dass diese in gewissen Anwendungsbereichen an ihre Leistungsgrenzen stoßen.

Gelenke im menschlichen Körper können gemäß ihren biomechanischen Freiheitsgraden sehr unterschiedliche physiologische Bewegungsfreiräume aufweisen.

Beispielsweise ist das Kniegelenk ein aus einem Dreh- und einem Scharniergelenk kombiniertes Gelenk, das zwei Freiheitsgrade in Form von Flexion/Extension und Rotation aufweist. Hingegen ist beispielsweise das obere Sprunggelenk ein reines Scharniergelenk, das nur einen Freiheitsgrad in Form einer Flexion des Fußes aufweist. Das Kniegelenk kann darüber hinaus als größtes Gelenk des menschlichen Körpers deutlich größere Kräfte aufnehmen als etwa das obere Sprunggelenk.

Aufgrund der starken biomechanischen Unterschiede verschiedener Gelenke des menschlichen Körpers kann es schwierig sein, Vorrichtungen so in ihrer Größe zu skalieren, dass die Ein- und Auszugsrichtung der geschwindigkeitsabhängig stabilisierenden Vorrichtung entlang der maximalen Abstandsänderung angeordnet werden kann. Darüber hinaus kann es der Fall sein, dass kleine Biegewinkel selbst bei unphysiologischer Bewegung keine hinreichende Längung erzeugen, die in eine wirksame Ein- bzw. Auszugsbewegung der geschwindigkeitsabhängig stabilisierenden Vorrichtung abgebildet werden kann. Gleichwohl kann bei Sportgeräten eine dämpfende Vorrichtung nicht immer entlang der maximalen Bewegungsänderung angeordnet werden, da häufig gerade die besonders beanspruchten Bereiche funktionale Wirkflächen mit spezifischen Eigenschaften darstellen.

Ein Beispiel für einen Anwendungsbereich derartiger Vorrichtigen sind Schuhsolen. Die Flexibilität von Schuhsolen richtet sich in der Regel primär nach den Materialeigenschaften. Dies führt dazu, dass Sohlen und damit Schuhe durch ihre vordefinierte Flexibilität auf einen gewissen Anwendungsbereich begrenzt sind. Da es aber auch Anwendungsbereiche gibt, für die die Anforderung an die Flexibilität der Schuhsole variieren kann, zum Beispiel Bergsteigen und Klettern in Kombination, besteht das Bedürfnis diesen wechselnden Anforderung mittels Bewegungsstabilisierungsvorrichtungen gerecht zu werden. Bekannte Vorrichtungen haben jedoch oftmals das Problem, dass sie ihre Wirkung in dem begrenzten Bauraum, den eine Schuhsole bereitstellt, nicht voll ausschöpfen können.

In der EP 2 984956 A1 ist eine Schuhsohle gemäß dem Oberbegriff des Anspruch 1 offenbart.

In der DE 10 2018 116569 B3 ist eine Vorrichtung zum Dämpfen einer Körperbewegung über ein Körpergelenk gezeigt.

DE 10 2017 112 911 A1 zeigt eine Gelenkeinrichtung.

WO 2018/020020 A1 zeigt eine Körpergelenkstabilisierungsvorrichtung zum adaptiven Dämpfen einer Körperbewegung.

In der DE 10 2017 117786 A1 ist ein Schuh zum Dämpfen einer Fußbewegung über das Sprunggelenk gezeigt.

US 2013/081304 A1 zeigt Sohlenkonstruktion für Energiespeicherung und Wiederabgabe.

US 5 103 811 A zeigt ein Körperteil oder Gelenkstütze.

In der EP 3 092 980 A1 ist eine Vorrichtung zur Stabilisierung von Körpergelenken, Muskeln und Sehnen gezeigt.

### Darstellung der Erfindung

Ausgehend von dem bekannten Stand der Technik ist es eine Aufgabe der vorliegenden Erfindung, eine Schuhsohle umfassend eine Vorrichtung zur Stabilisierung von Bewegungen der Schuhsohle anzugeben, welche die absolute Größe der relativen Bewegung der Schuhsohle von der Größe der Vorrichtung entkoppelt, so dass letztere weitgehend frei gestaltet werden kann. Nicht erfindungsgemäß, lediglich als Hintergrundinformation, kann die Vorrichtung auch an unterschiedlichen Gelenken und/oder in unterschiedlichen Sportgeräten verwendet werden.

Diese Aufgabe wird mittels einer Vorrichtung mit den Merkmalen des Anspruchs 1 gelöst. Vorteilhafte Weiterbildungen ergeben sich aus den Unteransprüchen.

Entsprechend wird eine Schuhsohle umfassend eine Vorrichtung zur Stabilisierung von Bewegungen zweier sich relativ zueinander bewegbarer Teile der Schuhsohle angegeben, umfassend eine an einem ersten Teil der Schuhsohle fixierbare Aufnahme, wobei die Aufnahme mit einem Füllmedium gefüllt ist, wobei die Aufnahme eine Aufnahmeöffnung umfasst, wobei die Vorrichtung ferner ein Kraftübertragungselement umfasst, und ein an einem zweiten Teil der Schuhsohle über das Kraftübertragungselement fixierbares Interaktionselement, das zumindest teilweise in der Aufnahme verschiebbar aufgenommen ist und sich durch die Aufnahmeöffnung erstreckt, wobei der Teil des Interaktionselements, der sich in der Aufnahme befindet, mit dem Füllmedium in Kontakt steht.

Erfindungsgemäß ist das Kraftübertragungselement schwenkbar an dem aus der Aufnahme ragenden Enden des Interaktionselements gehalten, wobei sich von der Aufnahme im Bereich der Aufnahmeöffnung mindestens ein Vorsprung in eine Auszugsrichtung des Interaktionselements, entlang der das Interaktionselement in der Aufnahme verschoben werden kann, erstreckt, wobei der Vorsprung mindestens einen ersten Kontaktbereich zum Kontaktieren des Kraftübertragungselements umfasst.

Dadurch ist die Steifigkeit der Schuhsohle abhängig von der Geschwindigkeit, mit der die Schuhsohle gekrümmt wird. Bei einer langsamen Krümmung, wie sie etwa beim Gehen auftritt, wird zwar das Interaktionselement über das Kraftübertragungselement gehebelt aus der Aufnahme heraus- und in die Aufnahme eingefahren, allerdings erfolgt die Verdrängung des Füllmediums durch das Interaktionselement derart langsam, dass die Vorrichtung nicht blockiert. In diesem Zustand ist die Schuhsohle flexibel. Bei einer schnellen Krümmung, wie sie etwa beim Rennen auftritt, ruft die Verdrängung des Füllmediums durch das Interaktionselement einen erhöhten Widerstand hervor, wodurch die Schuhsohle versteift. Durch die integrierte Vorrichtung kann die Schuhsohle eine geschwindigkeitsabhängige Stützwirkung entfalten.

Dieser Ansatz beruht auf der Erkenntnis, dass über eine Art Hebelfalle eine Vielzahl unterschiedlich orientierter Kraftvektoren, etwa aus einer Biegebewegung, durch Verkippung des Kraftübertragungselements eine Aktivierung der Vorrichtung ausgelöst werden kann.

Der Begriff "Körperbereich" ist als der Bereich zu verstehen, der die betreffenden, relativ zueinander bewegbaren Teile umfasst. Hinsichtlich des menschlichen Körpers muss ein Körperbereich im Sinne der vorliegenden Offenbarung demnach nicht zwangsläufig einem in der Anatomie begrifflich definierten Bereich des menschlichen Körpers entsprechen. Beispielsweise kann ein Körperbereich ein beliebig gewählter Teilbereich einer Hautoberfläche in der Nähe eines Gelenks sein. Denkbar ist aber ebenso, dass ein Körperbereich als ein Bereich definiert ist, der etwa eine Hand und einen Oberschenkel umfasst. Entscheidend ist, dass der Körperbereich Teile des menschlichen Körpers umfasst, die relativ zueinander bewegbar sind.

Ein Sportgerät im Sinne der vorliegenden Offenbarung ist eine Vorrichtung, die relativ zueinander bewegliche Teile oder Bereiche auffasst. Etwa kann dies ein Sportgerät sein, das bei Gebrauch im Wesentlichen die Bewegung des/der angrenzenden Gelenks/Gelenke nachbildet, insbesondere eine Sohle, ein Schuh, ein Stiefel, eine Bandage, eine Socke oder eine Orthese.

Die relativ zueinander beweglichen Teile müssen nicht zwangsläufig eine Bewegung des menschlichen Körpers nachbilden, sondern können alternativ oder zusätzlich eine sportgerättypische Bewegung abbilden, etwa eine Biege-, Torsions-, Kipp-, oder Rollbewegung. Beispielsweise kann das Sportgerät ein Ski oder ein Board sein.

Unter einem Füllmedium ist im Sinne der vorliegenden Offenbarung jedes Medium zu verstehen, das bei Verdrängung durch das Interaktionselement in der Aufnahme einen Widerstand gegenüber diesem ausbildet. Die Stoffeigenschaften des Mediums können vorzugsweise an eine gewünschte Widerstandscharakteristik angepasst sein, insbesondere eine geschwindigkeitsspezifische Widerstandscharakteristik. Beispielsweise kann das Füllmedium ein Fluid sein. Weiterhin kann die Aufnahme auch Wirkkörper umfassen die den Widerstand verstärken und/oder variieren bzw. adaptieren.

Die Einzugsrichtung sowie die Auszugsrichtung des Interaktionselements, entlang der das Interaktionselement in der Aufnahme verschoben werden kann, beziehen sich auf die Aufnahme, genauer gesagt auf die Wegänderung des Interaktionselements relativ zur Aufnahme. Wird also das Interaktionselement aus der Aufnahme heraus bewegt, wandert dieses in positiv definierter Richtung zumindest teilweise aus der Aufnahmeöffnung heraus. Gleichwohl wandert das Interaktionselement in negativ definierter Richtung zumindest teilweise in die Aufnahmeöffnung hinein, wenn das Interaktionselement in die Aufnahme hinein bewegt wird.

Grundsätzlich erfolgt das Betätigen der Vorrichtung über von außen auf das Kraftübertragungselement wirkende Kräfte und/oder Geschwindigkeiten, die das Kraftübertragungselement relativ zur Aufnahme beschleunigen. Eine Betätigung erfolgt aber frühestens dann, wenn die Beschleunigung des Kraftübertragungselements auch zu einer Beschleunigung des Interaktionselements auf eine voreingestellte Geschwindigkeit führt, die dann wiederum in der Aufnahme einen Widerstand generiert. Im trivialen Fall sind diese Kräfte parallel zur Auszugsrichtung bzw. Einzugsrichtung, was dazu führt, dass kein Kontakt zwischen dem Vorsprung und dem Kraftübertragungselement ausgebildet wird, und das Kraftübertragungselement das Interaktionselement in Auszugs- bzw. Einzugsrichtung belastet. Gleichwohl können die von außen anliegenden Kräfte nicht parallel sein und dennoch keinen Kontakt zwischen dem Kraftübertragungselement und dem Vorsprung ausbilden, wodurch das Kraftübertragungselement das Interaktionselement in Auszugs- bzw. Einzugsrichtung belastet. Die gehebelte Funktion erfordert mithin von außen am Kraftübertragungselement angreifende Kräfte, die eine Verkippung desselben in eine Richtung hervorrufen, in der ein Kontakt zwischen Kraftübertragungselement und Vorsprung geschaffen wird. Somit führt eine Kraft, die im Wesentlichen horizontal zur Längsachse des Kraftübertragungselements auf dieses wirkt, dazu, dass das Kraftübertragungselement hinzu dem Kontaktbereich des Vorsprungs bewegt wird, bis es an diesem anliegt. Durch den daraus resultierenden Hebel wird das Interaktionselement auch bei einer solchen Biegekraft in Auszugsrichtung ausgelenkt. Somit bleibt das Interaktionselement in Abhängigkeit der Geschwindigkeit beweglich oder wirkt stützend der Auslenkung entgegen.

Ein ganz wesentlicher Vorteil liegt darin, dass die Vorrichtung in Flächen eingearbeitet werden kann, wobei die Flächen bei Biegung oder Knicken relativ zueinander einen geschwindigkeitsabhängigen Widerstand in der Vorrichtung erzeugen. Dieser Widerstand bewirkt wiederum ein verändertes Biege- oder Knickverhalten der Flächen. Derartige Flächen können beispielsweise relativ zueinander bewegbare Teilflächen von Schuhsohlen, Skiern oder Boards sein. Erreicht eine Relativbewegung dieser Flächen eine vorab definierte Geschwindigkeit, kann diese Relativbewegung über einen geschwindigkeitsabhängigen Widerstand abgebremst werden. Genauer gesagt entstehen bei der Verdrängung des Füllmediums durch das Interaktionselement Reibungskräfte, die dann die Relativbewegung erschweren und Energie dissipieren. Mithin wird ein geschwindigkeitsabhängiger Widerstand erzeugt.

Ferner kann die Vorrichtung an einer ergonomisch unbedenklichen Stelle angeordnet werden. Das "Einfangen" oder "Ableiten" der Kraftvektoren erfolgt dabei über eine gezielte Anpassung des Vorsprungs bzw. des Kontaktbereichs. So kann die Vorrichtung gezielt dazu ausgelegt werden, eine Vielzahl von unterschiedlichen Kräften in eine Verkippung zu übersetzen. Im Ergebnis führt dies dazu, dass mit einer einzigen Vorrichtung ein breites Sicherheitsspektrum gegenüber vieler unterschiedlich gerichteter Kräfte realisiert werden kann.

Um jene Hebelwirkung hervorrufen zu können, muss das Kraftübertragungselement hinreichend biegesteif sein. Für die Ausgestaltung des an einem zweiten Teil desselben menschlichen Körperbereichs und/oder desselben Sportgeräts fixierbaren Interaktionselements sowie die schwenkbare Halterung des Kraftübertragungselements an dem Interaktionselement sind hingegen unterschiedliche Ausgestaltungsvarianten denkbar. Beispielsweise kann bei entsprechender Halterung am Kraftübertragungselement das Interaktionselement ein biegesteifes oder elastisches Bauteil sein. Entscheidend für die beabsichtigte Funktion der Vorrichtung ist lediglich, dass ein Verkippen des Kraftübertragungselements über den Kontaktbereich zu einer Verschiebung des Teils des Interaktionselements erfolgt, der sich innerhalb der Aufnahme befindet.

Der Vorsprung bildet eine Stützstruktur für das Kraftübertragungselement. Der Vorsprung kann stabförmig, plattenförmig oder rohrförmig sein, oder andere Geometrien umfassen, um die zu übertragenden Kraftflüsse vom Kontaktbereich in die Aufnahme zu leiten. Beispielsweise kann der Vorsprung einen oder mehrere Stege aufweisen oder etwa als Hülse ausgeführt sein. Neben dem Bereitstellen des Kontaktbereichs muss der Vorsprung so dimensioniert sein, dass dieser hinreichend biegesteif ist, um Kräfte aus dem Kraftübertragungselement so aufzunehmen, dass diese über die Hebelwirkung am Kontaktbereich zu einer Auslenkung des Interaktionselements führen. Dabei ist vorteilhaft, die zu stützenden Verkippungsrichtungen des Kraftübertragungselements über einen individuell bestimmten Abstand des Kontaktbereichs zur Aufnahmeöffnung in eine Auslenkung des Interaktionselements zu übersetzen.

Durch den Kontakt des Teils des Interaktionselements, der sich in der Aufnahme befindet, mit dem Füllmedium kann ein unmittelbarer Kraftfluss zwischen Füllmedium und Interaktionselement hergestellt werden. Der Vorteil ist dabei, dass die Vorrichtung zuverlässig und vorhersagbar alle Kräfte, die über das Interaktionselement in die Aufnahme eingetragen werden, allein über die geschwindigkeitsabhängigen Widerstandsänderungen des Füllmediums dämpft oder nicht dämpft. Der besagte Teil des Interaktionselements steht auch dann im mit dem Füllmedium in Kontakt, wenn jener Teil des Interaktionselements eine Beschichtung, eine Umhüllung oder ein Zwischenelement aufweist. Vorteilhaft ist, dass jede Verschiebung des Interaktionselements entlang der Einzugs- und Auszugsrichtung in einer Wechselwirkung zwischen Interaktionselement und Füllmedium resultiert.

In einer vorteilhaften Ausführungsform umfasst der Vorsprung mindestens einen zweiten Kontaktbereich zum Kontaktieren des Kraftübertragungselements. Der mindestens eine zweite Kontaktbereich liegt dem mindestens einen ersten Kontaktbereich in einer zur Auszugsrichtung orthogonalen Ebene und in Bezug zur Auszugsrichtung gegenüber. Dies hat den Vorteil, dass zwei verschiedene Verkippungsrichtungen, in welche das Kraftübertragungselement bewegt werden kann, vorliegen können. Bevorzugt liegt der mindestens eine zweite Kontaktbereich dem mindestens einem ersten Kontaktbereich in einer zur Auszugsrichtung orthogonalen Ebene und in Bezug zur Auszugsrichtung gegenüber.

Ein "Gegenüberliegen" im Sinne der vorliegenden Offenbarung bedeutet, dass der erste und zweite Kontaktbereich in einer gedachten Ebene liegen, die orthogonal zur Auszugsrichtung konstruiert ist, wobei sich die Kontaktbereiche auch bezüglich einer zwischen ihnen verlaufenden Auszugsrichtung gegenüberliegen. "Gegenüberliegen" umfasst auch, dass der erste und zweite Kontaktbereich in unterschiedlichen Ebenen liegen, die beide orthogonal zur Auszugsrichtung verlaufen, etwa wenn die Kontaktbereiche in unterschiedlichen Abständen zur Aufnahmeöffnung angeordnet sind.

Das Vorsehen eines derartigen zweiten Kontaktbereichs hat den Vorteil, dass die Vorrichtung beispielsweise in einem rechten Winkel zu einer auf die Vorrichtung einwirkenden Kraft angeordnet werden kann. Dadurch kann der für die Vorrichtung erforderliche Bauraum reduziert werden und die Vorrichtung muss nicht entlang des Kraftvektors einer auf die Vorrichtung wirkenden Kraft installiert werden muss. Mithin kann ein Großteil der Vorrichtung an einer Stelle außerhalb des Kraftflusses in einem Körperbereich oder einem Sportgerät positioniert werden.

Alternativ oder zusätzlich können ein oder mehrere Vorsprünge Kontaktbereiche aufweisen, die einen unterschiedlichen Abstand zur Aufnahmeöffnung aufweisen. Beispielsweise kann der Abstand zwischen Aufnahmeöffnung und Kontaktbereich eine Funktion der Umfangskoordinate der Aufnahmeöffnung sein. Die Funktion kann stetig und/oder unstetig sein. Dadurch können unterschiedliche Kräfte hinsichtlich ihrer erwarteten Richtung und Größe individuellen Hebeln zugewiesen werden, was sich vorteilhaft auf die Sicherheit und Ergonomie der Vorrichtung auswirkt.

Gemäß einer vorteilhaften Weiterbildung umfasst die Vorrichtung weiter ein erstes Anbindungselement zum Verbinden des Interaktionselements mit dem Kraftübertragungselement, wobei das Anbindungselement bevorzugt eine Öse oder ein Gelenk, bevorzugt ein Drehgelenk oder ein Kugelgelenk, umfasst, wobei bevorzugt das Anbindungselement integral mit dem Interaktionselement oder dem Kraftübertragungselement ausgebildet ist. Die Verwendung einer Öse oder eines Gelenks ermöglicht, dass eine auf das Kraftübertragungselement einwirkende Kraft das Kraftübertragungselement relativ zum Interaktionselement schwenken kann, und bis zu einem Kontakt mit dem Kontaktbereich auf dem Vorsprung zubewegt werden kann. Dadurch kann aufgrund des daraus resultierenden Hebels das Interaktionselement auch bei einer Biegekraft in Auszugsrichtung ausgelenkt werden.

Insbesondere wenn nicht nur das Kraftübertragungselement, sondern nun auch das Interaktionselement im Wesentlichen biegesteif sind, können Verkippungen des Kraftübertragungselements relativ zum Interaktionselement in eine oder mehrere Richtungen realisiert werden. Zusätzlich kann die Öse und/oder das Gelenk so ausgebildet sein, dass diese bzw. dieses entlang des mindestens einen Vorsprungs geführt wird. Die Verwendung einer Öse und/oder eines Gelenks hat weiterhin den Vorteil, dass die Montage und/oder eine Reparatur der Vorrichtung dadurch erleichtert wird. Beispielsweise kann das Kraftübertragungselement lösbar über die Öse und/oder das Gelenk am Interaktionselement befestigt sein.

In einer Weiterbildung umfasst das Anbindungselement ein Drehgelenk oder ein Kugelgelenk. Mithilfe derartiger Gelenke können mehrere Verkippungsrichtungen des Kraftübertragungselements gestützt werden. Alternativ kann das Anbindungselement integral mit dem Interaktionselement oder dem Kraftübertragungselement ausgebildet sein. Das Kraftübertragungselement kann eine hinreichende Biegesteifigkeit aufweist, um die Hebelwirkung am Kontaktbereich des Vorsprung hervorzurufen. Das Interaktionselement selbst hingegen kann auch ein elastischer Körper sein und in diesem Falle integral mit dem Kraftübertragungselement verbunden sein. Dadurch können einfache und sichere Bauteilverbindungen realisiert und zudem Herstellungskosten eingespart werden. Der Begriff integral umfasst sowohl einstückige Bauteile als auch Bauteile, die aus mehreren Teilen zu einem Bauteilverbund zusammengesetzt wurden. Beispiel für solch einen Bauteilverbund sind etwa Klebeverbindungen aber auch Schweiß-, Schraub-, Klemm-, Pressverbindungen oder dergleichen.

In einer bevorzugten Weiterbildung umfasst der mindestens eine erste Kontaktbereich mindestens einen Schwenkkopf, wobei das Kraftübertragungselement den mindestens einen Schwenkkopf kontaktieren und um diesen schwenken kann, wobei zumindest ein Teil des mindestens einen Schwenkkopfs einen kreisförmigen oder elliptischen Abschnitt zum Kontaktieren des Kraftübertragungselements umfasst.

Wie vorstehend beschrieben, sind die mögliche Auslenkung bzw. der Hub des Interaktionselements unter anderem von der Kontur des Kontaktbereichs abhängig. Ein Schwenkkopf am Kontaktbereich ermöglicht eine Vergrößerung des Kontaktbereichs dahingehend, dass das Kraftübertragungselement schon bei kleineren Verkippungen in Kontakt mit dem Kontaktbereich kommt. Anders formuliert weist der mindestens eine Schwenkkopf einen Radius auf. Dadurch kann gewährleistet werden, dass die Vorrichtung auch bei kleineren Verkippungen, bzw. Biege-Winkeln, sensibler reagiert. Der für die Funktion der Vorrichtung notwendige Raum kann dadurch reduziert werden. Grundsätzlich ist es vorteilhaft, die Vorrichtung so flach und klein wie möglich auszubilden, so dass sie einfach integrierbar ist und den Anwender möglichst nicht behindert. Gleiches gilt auch für den Schwenckopf bzw. die Schwenkköpfe. Ferner ist es vorteilhaft, die Vorrichtung so stabil wie möglich auszugestalten, damit sich ein Kraftfluss einstellt, über den von außen am Kraftübertragungselement angreifende Kräfte in eine Wechselwirkung mit dem Füllmedium gezwungen werden.

Der mindestens eine Schwenkkopf kann dabei entlang des gesamten Kontaktbereichs oder nur entlang eines Teils des Kontaktbereichs ausgebildet sein. Sofern mehrere Kontaktbereiche vorliegen, etwa wenn mehrere separate Vorsprünge vorhanden sind, kann an allen oder nur an manchen Kontaktbereichen ein Schwenkkopf vorgesehen sein. Der zumindest eine Teil des Schwenkkopfs, der einen kreisförmigen oder elliptischen Abschnitt zum Kontaktieren des Kraftübertragungselements aufweist, kann beispielsweise als eine Wulst ausgebildet sein, die den Kontaktbereich bildet. Der mindestens eine Schwenkkopf kann elastisch verformbar oder starr sein. Für die Auslegung des mindestens einen Schwenkkopfs gelten im Grunde die gleichen Voraussetzungen wie für die Auslegung der ersten und/oder zweiten Kontaktbereiche als solche.

In einer Weiterbildung umfasst die Vorrichtung ferner ein zweites Anbindungselement zum Verbinden des Kraftübertragungselements mit dem zweiten Teil des menschlichen Körperbereichs und/oder des Sportgeräts, wobei das zweite Anbindungselement bevorzugt eine Öse oder ein Gelenk, ein Drehgelenk oder ein Kugelgelenk umfasst. Dies ist insbesondere dann von Vorteil, wenn der zweite Teil des menschlichen Körperbereichs und/oder Sportgeräts eine Fläche aufweist, die bezüglich mehrerer Verkippungsrichtungen des Kraftübertragungselements gestützt werden soll. Mithilfe des zweiten Anbindungselements kann beispielsweise ein zweites Kraftübertragungselement vorgesehen sein, das an einer zweiten Position des zweiten Körpers verbunden ist. Im Ergebnis kann die Vorrichtung dazu verwendet werden, ganze Flächen geschwindigkeitsabhängig zu stabilisieren. Die zuvor geschilderten Vorteile der Verwendung einer Öse oder eines Gelenks gelten hier entsprechend.

Analog zu den oben genannten Ausführungen kann auch das zweite Anbindungselement gemäß einer bevorzugten Weiterbildung ein Drehgelenk oder ein Kugelgelenk umfassen. Wie zuvor geschildert hat die Verwendung eines Drehgelenks oder eines Kugelgelenks den Vorteil, dass verschiedene Verkippungsrichtungen des Kraftübertragungselements gestützt werden können.

In einer weiter bevorzugten Ausführungsform umfasst das Kraftübertragungselement einen Endanschlag zum Kontaktieren des mindestens einen ersten Kontaktbereichs, um einen Verschiebeweg des Kraftübertragungselements in Richtung der Aufnahme zu begrenzen.

Dadurch kann das Kraftübertragungselement gegenüber der Aufnahme abgestützt werden. Ferner fahren die relativ zueinander bewegten Komponenten der Vorrichtung nicht übermäßig aufeinander, bzw. die Vorrichtung wird nicht gestaucht. Ein weiterer Vorteil ist, dass auch beim Verrutschen der Vorrichtung eine Abstützung sichergestellt werden kann. So kann vermieden werden, dass selbst bei unsachgemäßer Flexion oder Extension des ersten und zweiten Teils eines menschlichen Körperbereichs und/oder eines Sportgeräts zueinander ein Schaden an der Vorrichtung auftritt. Der Endanschlag kann plan, konkav oder konvex ausgeführt sein. Der Endanschlag kann jedoch auch eine ebene Fläche aufweisen. Zudem kann der Endanschlag Eigenschaften aufweisen, die ein Abgleiten des Endanschlags auf dem Kontaktbereich fördern. Die Bereiche vorteilhafter Reibwerte bestimmen sich nach DIN EN ISO 8295. Dadurch ist es möglich, den Widerstand, welcher auf die Gleitreibung zwischen dem Endanschlag und dem Kontaktbereich zurückzuführen ist, gering zu halten, wodurch der normale Bewegungsablauf nicht durch das Vorhandensein einer erfindungsgemäßen Vorrichtung beeinträchtigt wird.

Beispielweise kann ein Schwenkkopf am Vorsprung angeordnet sein, wobei der Schwenkkopf einen ersten Kontaktbereich für das Kraftübertragungselement ausbildet und zusätzlich den Endanschlag kontaktieren kann. In diesem Fall ist der Begriff "Kontaktbereich" so zu verstehen, dass auch Kontaktpunkte des Endanschlags mit umfasst sind.

Gemäß einer bevorzugten Weiterbildung weist der Endanschlag eine in Bezug auf die Aufnahme konkave Oberfläche auf, deren Innenradius in etwa so groß ist wie der Abstand des Endanschlags zum Ende des Kraftübertragungselements, das zwischen Endanschlag und Aufnahme liegt. Dies begünstigt das Abgleiten des Schwenkkopfes bzw. der Schwenkköpfe auf der Innenseite des Endanschlags sowie Gelenkbewegungen wie etwa Flexion und Extension oder Adduktion und Abduktion.

Gemäß einer weiteren bevorzugten Weiterbildung weist der Endanschlag einen halbmondförmigen Querschnitt auf. Dies hat den Vorteil, dass zwischen dem Kontaktbereich und der Innenfläche des halbmondförmigen Querschnitts ein besonders gutes Abgleiten möglich ist, wodurch Reibungswiderstände reduziert werden, wenn der Endanschlag mit dem Kontaktbereich einen Anschlag ausbildet.

In einer weiter bevorzugten Ausgestaltung umfasst der Vorsprung eine Einhausung, wobei die Einhausung zumindest einen Teil des sich aus der Aufnahmeöffnung der Aufnahme erstreckenden Interaktionselements umgibt. Vorteilhaft ist dabei, dass die Einhausung den für die Funktion der Vorrichtung besonders sensiblen Bereich, nämlich den Übergang zwischen Interaktionselement und Kraftübertragungselement nach außen hin schützt. Gleichzeitig kann die Innenseite der Einhausung so ausgebildet sein, dass das Interaktionselement und das Kraftübertragungselement und gegebenenfalls das Anbindungselement gleitfördernd an einer Innenwand der Einhausung geführt werden können. Auch kann über eine derartige Einhausung sichergestellt werden, dass übermäßige Kräfte, die auf das Kraftübertragungselement in die Vorrichtung eingeleitet werden, nicht zu einer Beschädigung der Vorrichtung führen, da die Einhausung einen mittelbaren Schutz der relativ zur Aufnahme bewegten Teile bietet.

Alternativ oder ergänzend kann die Einhausung auch eine Sollbruchstelle umfassen, die verhindert, dass übermäßige auf das Kraftübertragungselement wirkende Kräfte in die Aufnahme eingeleitet werden. Ferner können der eine oder die mehreren Kontaktbereiche an der Einhausung angeordnet sein. Die Einhausung kann insbesondere eine Hülse sein.

Gemäß einer weiteren Ausgestaltung umfasst die Einhausung ein Sichtfenster. Über das Sichtfenster kann die Vorrichtung von außen auf ihre Funktion hin überprüft werden. Gleichwohl können informative und/oder ästhetische Effekte erzielt werden. Insbesondere kann über ein Sichtfenster die Funktion der Vorrichtung visuell nachvollzogen werden, was beispielsweise für Marketingzwecke genutzt werden kann. Gleichzeitig könnte ein derartiges Sichtfenster auch als Schnittstelle für weitere informationsverarbeitende Einrichtungen genutzt werden. Beispielsweise kann über das Sichtfenster die Auslenkung relativ bewegter Komponenten der Vorrichtung aufgezeichnet und ausgewertet werden.

Gemäß einer weiteren Ausgestaltung erstreckt sich der Vorsprung konisch in die Auszugsrichtung des Interaktionselements, derart, dass der Abstand zwischen einer Längsachse des Interaktionselements und dem Vorsprung in Auszugsrichtung abnimmt oder zunimmt. Die Längsachse des Interaktionselements ist als gedachte Verlängerung der in Auszugsrichtung verlaufenden Körperachse des Interaktionselements zu verstehen. Maßgeblich für die Definition des Abstands ist dabei die Innenseite des Vorsprungs.

Dadurch kann die Hebelwirkung begünstigt werden. Genauer gesagt kann dadurch - ähnlich wie mittels des Schwenkkopfes bzw. der Schwenkköpfe - der Kontaktbereich näher oder entfernter zum Kraftübertragungselement angeordnet werden. Darüber kann die Hebelwirkung über einen zusätzlichen Parameter, nämlich die Neigung des Vorsprungs, gezielt eingestellt werden.

Weiterhin hat die Abnahme des Abstands zwischen einer Längsachse des Interaktionselements und dem Vorsprung in Auszugsrichtung den Vorteil, dass dadurch der Kraftfluss im vorsprungsseitigen Kontaktpunkt in den Vorsprung optimiert wird. Die im Kontakt mit dem Kraftübertragungselement im Kontaktbereich auftretenden Kräfte können bei dieser Ausgestaltungsform verstärkt in Normalenrichtung angreifen, wodurch auf den Vorsprung mehr Druck- als Biegekräfte wirken.

Gemäß einer bevorzugten Ausführungsform umfasst die Vorrichtung ein Umlenkelement zum Umlenken einer auf das Kraftübertragungselement wirkenden Kraft und ein Verbindungselement, wobei das Umlenkelement an dem Kraftübertragungselement angeordnet ist, wobei das Interaktionselement mit dem einen Ende des Verbindungselements und der mindestens eine Vorsprung oder die Aufnahme mit dem anderen Ende des Verbindungselements verbunden sind, und wobei das Verbindungselement über das Umlenkelement am Interaktionselement geführt ist. Alternativ kann das andere Ende des Verbindungselements statt mit dem Vorsprung auch unmittelbar mit der Aufnahme verbunden sein.

Durch die vorstehend beschriebene Anordnung kann eine Hubverdopplung innerhalb der Vorrichtung realisiert werden. Je nachdem in welcher Reihenfolge das Interaktionselement mit dem Kraftübertragungselement und dem Umlenkelement mittels des Verbindungselements miteinander verbunden sind, können unterschiedliche Kraftwandlungsszenarien bereitgestellt werden. Somit ist es möglich den Umfang und die Intensität einer von einem Körperteil ausgehenden Bewegung gegenüber dem Bewegungsumfang und der Bewegungsintensität des Kraftübertragungselements je nach Anwendungsbereich zu vergrößern oder zu reduzieren. Dadurch können große Vorrichtungen auch zum Dämpfen von kleinen relativen Körperbewegungen eingesetzt werden.

Umgekehrt können kleine Vorrichtungen dadurch auch bei größeren relativen Körperbewegungen zum Einsatz kommen. Insgesamt lässt sich die absolute Größe der relativen Bewegung der Körperteile von der Größe der Vorrichtung entkoppeln. Dadurch kann das Einsatzgebiet der Vorrichtung deutlich vergrößert werden. Darüber hinaus ergeben sich zusätzliche Freiheiten in Bezug auf die Gestaltung der Vorrichtung, da diese weniger stark vom Einsatzgebiet der Vorrichtung beeinflusst wird.

Auf diese Art und Weise können selbst kleine Auslenkungen des Kraftübertragungselements, die ohne Hubverstärkung zu keiner merklichen Stabilisierung durch die Vorrichtung führen, verstärkt, insbesondere verdoppelt, werden. Darüber hinaus können selbst solche Kräfte verstärkt werden, die gar keine Verkippung des Kraftübertragungselements hervorrufen würden, beispielsweise parallel zur Auszugs- bzw. Einzugsrichtung wirkende Kräfte.

Dadurch kann die Vorrichtung beispielsweise in einer Knie-Bandage oder einer Orthese eingesetzt werden, die zum einen diverse Gelenksbewegungen des Kniegelenks zulassen kann, sofern diese im physiologischen Bereich liegen, und zum anderen bei Erreichen einer vorab definierten Geschwindigkeit und/oder unvorteilhaften Winkeländerungen, die zu einer Verletzung führen könnten, entsprechend abstützen.

Der Vorteil einer solchen Hubverstärkung kommt insbesondere bei dem Bestreben, sehr kleine Winkeländerungen zu dämpfen, zum Tragen. Etwa besteht bei der Verletzungsprävention von Sportlern seit jeher der Wunsch, das Kniegelenk gegen mediolateral induzierte Überbeanspruchungen zu schützen. Der hierfür notwendige Arbeitsweg eines Dämpfers muss jedoch im Bereich weniger Millimeter liegen, um überhaupt einen Schutz zu entfalten. Dies kann mit einer Hubvervielfachung erzielt werden.

Die Aufgabe wird ferner mit einem Schuh, in den die vorstehende Vorrichtung integriert ist, gelöst. Beispielsweise kann die Vorrichtung im Wesentlichen vertikal in einen Stiefel integriert sein, wobei die Vorrichtung in eine Sohle des Schuhs integriert ist. Dadurch kann der Fersenbereich der Sohle gegenüber dem Ballenbereich der Sohle bei einer unphysiologischen Relativbewegung abgestützt werden.

Mittels eines derartigen Schuhs können unphysiologische Bewegungsgeschwindigkeiten über einen geschwindigkeitsabhängigen Dämpfer in Form einer zuvor beschriebenen Vorrichtung dissipiert werden und so die Gefahr von Verletzungen reduziert werden.

In einer weiteren beispielhaften Ausführungsform kann die Schuhsohle mindestens zwei Bereiche unterschiedlicher Flexibilität aufweisen. Dadurch kann die Schuhsole zusätzlichen Anforderungsprofilen gerecht werden.

### Kurzbeschreibung der Figuren

Bevorzugte weitere Ausführungsformen und Aspekte der vorliegenden Erfindung werden durch die nachfolgende Beschreibung der Figuren näher erläutert. Dabei zeigen:
- Figuren 1A-1D: eine erste Ausführungsform einer Vorrichtung in einer Ausgangsposition und einer Position, in der das Kraftübertragungselement am Kontaktbereich anliegt, jeweils als Schnittzeichnung und in perspektivischer Darstellung;
- Figuren 2A-2D: eine zweite Ausführungsform einer Vorrichtung in einer Ausgangsposition und einer Position, in der ein Kraftübertragungselement an einem Kontaktbereich anliegt, jeweils als Schnittzeichnung und in perspektivischer Darstellung;
- Figuren 3A-3D: eine dritte Ausführungsform einer Vorrichtung in einer Ausgangsposition und einer Position, in der ein Kraftübertragungselement an einem Kontaktbereich anliegt, jeweils als Schnittzeichnung und in perspektivischer Darstellung;
- Figuren 4A-4D: eine vierte Ausführungsform einer Vorrichtung in einer Ausgangsposition und einer Position, in der ein Kraftübertragungselement an einem Kontaktbereich anliegt, jeweils als Schnittzeichnung und in perspektivischer Darstellung;
- Figuren 5A-5B: eine fünfte Ausführungsform einer Vorrichtung in einer Ausgangsposition und einer Position, in der ein Kraftübertragungselement an einem Kontaktbereich anliegt, jeweils als Schnittzeichnung;
- Figuren 6A-6B: eine sechste Ausführungsform einer Vorrichtung in einer Ausgangsposition und einer Position, in der ein Endanschlag an einem Schwenkkopf anliegt, jeweils als Schnittzeichnung;
- Figuren 7A-7B: eine siebte Ausführungsform einer Vorrichtung in einer Ausgangsposition und einer Position, in der ein Kraftübertragungselement an einem Kontaktbereich anliegt, jeweils als Schnittzeichnung;
- Figuren 8A-8B: eine achte Ausführungsform einer Vorrichtung in einer Ausgangsposition und einer Position, in der ein Kraftübertragungselement an einem Kontaktbereich anliegt, jeweils als Schnittzeichnung;
- Figuren 9A-9C: eine neunte Ausführungsform einer Vorrichtung in einer Ausgangsposition und einer Position, in der ein Kraftübertragungselement an einem Kontaktbereich anliegt, als Schnittzeichnung und einer perspektivischen Darstellung der Ausgangsposition;
- Figuren 10A-10C: eine zehnte Ausführungsform einer Vorrichtung in einer Ausgangsposition und einer Position, in der ein Kraftübertragungselement an einem Kontaktbereich anliegt, als Schnittzeichnung und einer perspektivischen Darstellung der Ausgangsposition;
- Figur 11: schematisch eine Seitenansicht eines nicht erfindungsgemäßen Schuhs mit einer Vorrichtung gemäß der sechsten Ausführungsform in einer Ausgangsposition;
- Figur 12: schematisch eine Seitenansicht einer nicht erfindungsgemäßen Kniemanschette an einem menschlichen Knie mit einer Vorrichtung gemäß der sechsten Ausführungsform in einer Ausgangsposition;
- Figur 13A: schematisch eine Draufsicht von unten auf eine Schuhsohle mit einer Vorrichtung gemäß der dritten Ausführungsform in einer Ausgangsposition; und
- Figur 13B: schematisch eine seitliche Schnittansicht einer Schuhsohle mit einer Vorrichtung gemäß der dritten Ausführungsform in einer Ausgangsposition.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

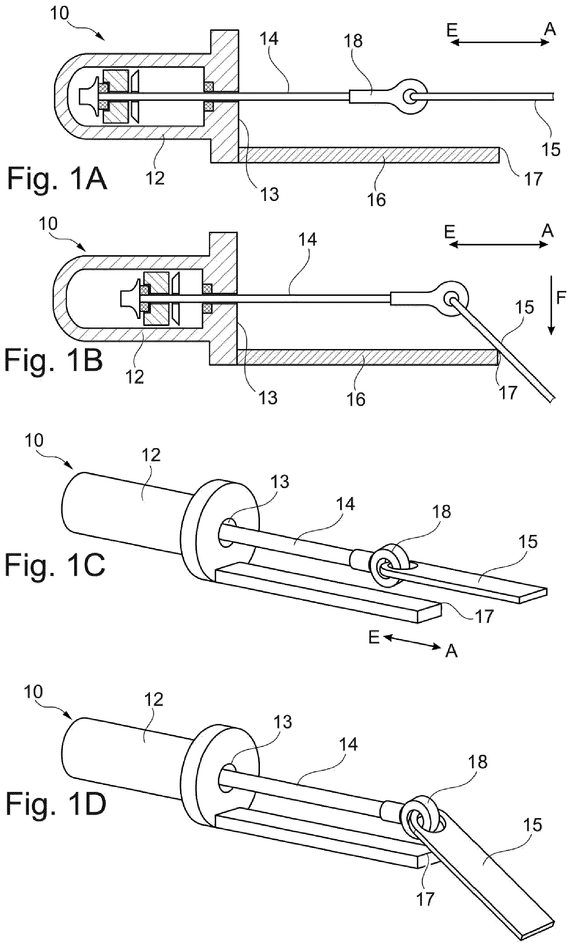

Figuren 1A und 1C zeigen eine erste Ausführungsform der Vorrichtung 10 in einer Ausgangsposition. Die Ausgangsposition der Vorrichtung 10 ist in Figur 1A als Schnittdarstellung und in Figur 1C als perspektivische Darstellung gezeigt.

Gezeigt ist eine an einem nicht abgebildeten ersten Teil eines menschlichen Körperbereichs und/oder eines Sportgeräts fixierbare Aufnahme 12, die mit einem Füllmedium gefüllt ist. Die Aufnahme 12 umfasst dabei eine Aufnahmeöffnung 13 sowie ein an einem nicht abgebildeten zweiten Teil desselben menschlichen Körperbereichs und/oder desselben Sportgeräts fixierbares Interaktionselement 14, das zumindest teilweise in der Aufnahme 12 verschiebbar aufgenommen ist und sich durch die Aufnahmeöffnung 13 erstreckt. Der Teil des Interaktionselements 14, der sich in der Aufnahme 12 befindet, steht dabei in Kontakt mit dem Füllmedium.

An der Vorrichtung 10 ist ferner ein Kraftübertragungselement 15 angeordnet, das schwenkbar an dem aus der Aufnahme 12 ragenden Ende des Interaktionselements 14 gehalten ist. Von der Aufnahme 12 erstreckt sich im Bereich der Aufnahmeöffnung 13 ein Vorsprung 16 in eine Auszugsrichtung A des Interaktionselements 14, entlang der das Interaktionselement 14 in der Aufnahme 12 verschoben werden kann. Der Vorsprung 16 umfasst einen Kontaktbereich 17 zum Kontaktieren des Kraftübertragungselements 15. In der in den Figuren 1A und 1C abgebildeten Ausgangsposition der Vorrichtung 10 bildet das Kraftübertragungselement 15 keinen Kontakt mit dem Kontaktbereich 17 aus. Sofern Kräfte, die von außen auf das Kraftübertragungselement 15 aufgebracht werden, zu einer Verschiebung des Kraftübertragungselements 15 führen, wird diese Verschiebung über das Anbindungselement 18 auf das Interaktionselement 14 übertragen. In der Ausgangsposition ist die Verschiebung des Kraftübertragungselements 15 identisch zur Verschiebung des Interaktionselements 14 entlang der Auszugsrichtung A bzw. Einzugsrichtung E.

Der Teil des Interaktionselements 14, der sich in der Aufnahme 12 befindet, interagiert mit dem Füllmedium, was bei einer unphysiologischen Einzugs- bzw. Auszugsgeschwindigkeit des Interaktionselements 14 relativ zur Aufnahme 12 einen Widerstand in der Aufnahme 12 hervorruft. Dadurch wird eine Bewegung des Kraftübertragungselements 15 in Einzugsrichtung E bzw. Auszugsrichtung A geschwindigkeitsabhängig gebremst bzw. unterbunden.

Figuren 1B und 1D zeigen die erste Ausführungsform der Vorrichtung 10 in einer Position, in der das Kraftübertragungselement 15 an dem Kontaktbereich 17 anliegt. Figur 1B zeigt diese Position anhand einer Schnittdarstellung und Figur 1D zeigt diese Position anhand einer perspektivischen Darstellung.

Diese Position wird erreicht, wenn an dem Kraftübertragungselement 15 eine Kraft F angreift, die ausreichend groß ist, um eine Verkippung des Kraftübertragungselements 15 bis zum ersten Kontaktbereich 17 hervorzurufen. Führt die Kraft F zu einer weiteren Auslenkung des Kraftübertragungselements 15, zieht dieses das Interaktionselement 14 über das Anbindungselement 18 aus der Aufnahme 12 heraus, wobei das Herausziehen in Auszugsrichtung A über den ersten Kontaktbereich 17 gehebelt erfolgt. Mithin zeigen die Figuren 1D und 1B das Interaktionselement 14 in einer Position, die gegenüber der in den Figuren 1A und 1C gezeigten Position einer herausgezogenen Position entspricht.

Durch den Kontakt des Kraftübertragungselements 15 am Kontaktbereich 17 und durch eine am Kraftübertragungselement 15 angreifende Kraft F findet eine Verkippung des Kraftübertragungselements 15 statt. Diese Verkippung resultiert in einer gehebelten Verschiebung des Interaktionselements 14, das über das Anbindungselement 18 mit dem Interaktionselement 14 verbunden ist.

Sofern das Interaktionselement 14 dabei eine ausreichende Geschwindigkeit relativ zur Aufnahme 12 erreicht, wird dieses geschwindigkeitsabhängig über den füllmediuminduzierten Widerstand in der Aufnahme 12 gebremst. Die Kraftübertragung zwischen Füllmedium und Interaktionselement 14 erfolgt über den innerhalb der Aufnahme 12 befindlichen Teil des Interaktionselements 14. Diese Verzögerung wird von dem Interaktionselement 14 über das Anbindungselement 18 an das Kraftübertragungselement 15 rückgekoppelt, wodurch eine Relativbewegung des ersten Teils eines menschlichen Körperbereichs und/oder Sportgeräts (nicht abgebildet) relativ zu einem zweiten Teil desselben menschlichen Körperbereichs und/oder desselben Sportgeräts gestützt werden kann.

In der in den Figuren 1A bis 1D offenbarten Ausführungsform ist das Anbindungselement 18 als eine Öse ausgeführt, die ein Schwenken des Kraftübertragungselements 15 von der Ausgangsposition in die kontaktierende Position ermöglicht. Alternativ kann das Kraftübertragungselement 15 auch direkt mit dem Interaktionselement 14 verbunden sein.

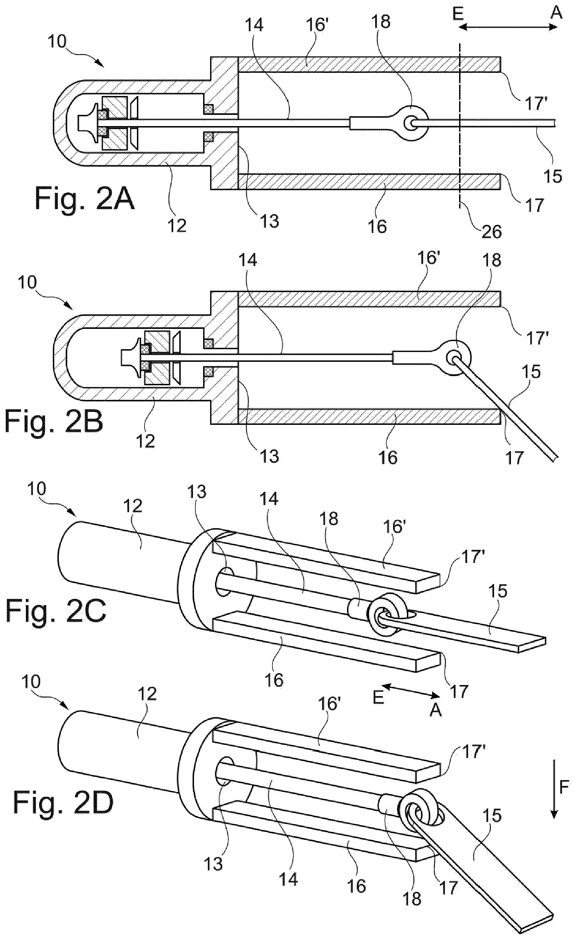

Figuren 2A und 2C zeigen eine zweite Ausführungsform der Vorrichtung 10 in einer Ausgangsposition. Die Ausgangsposition der Vorrichtung 10 ist in Figur 2A als Schnittdarstellung und in Figur 2C als perspektivische Darstellung gezeigt.

Die zweite Ausführungsform unterscheidet sich von der ersten Ausführungsform dadurch, dass neben dem ersten Vorsprung 16 zusätzlich ein zweiter Vorsprung 16`, vorhanden ist, welcher einen zweiten Kontaktbereich 17' zum Kontaktieren des Kraftübertragungselements 15 aufweist. Der zweite Kontaktbereich 17' liegt dabei dem ersten Kontaktbereich 17 in einer zur Auszugsrichtung A orthogonalen Ebene 26 und in Bezug zur Auszugsrichtung A gegenüber. Der erste Vorsprung 16 und der zweite Vorsprung 16' sind in der zweiten Ausführungsform jeweils stegförmig ausgebildet.

Figuren 2B und 2D zeigen die zweite Ausführungsform der Vorrichtung 10 in einer Position, in der das Kraftübertragungselement 15 an dem Kontaktbereich 17 anliegt. Figur 2B zeigt diese Position anhand einer Schnittdarstellung und Figur 2D zeigt diese Position anhand einer perspektivischen Darstellung.

Führt die Kraft F zu einer weiteren Auslenkung des Kraftübertragungselements 15, zieht dieses das Interaktionselement 14 über das Anbindungselement 18 aus der Aufnahme 12 heraus, wobei das Herausziehen in Auszugsrichtung A über entweder den ersten Kontaktbereich 17 bzw. den zweiten Kontaktbereich 17' gehebelt erfolgt. In den Darstellungen der Figuren 2B und 2D liegt das Kraftübertragungselement 15 am ersten Kontaktbereich 17 an. Mithin zeigen die Figuren 2D und 2B das Interaktionselement 14 in einer Position, die gegenüber der in den Figuren 2A und 2C gezeigten Position einer herausgezogenen Position entspricht.

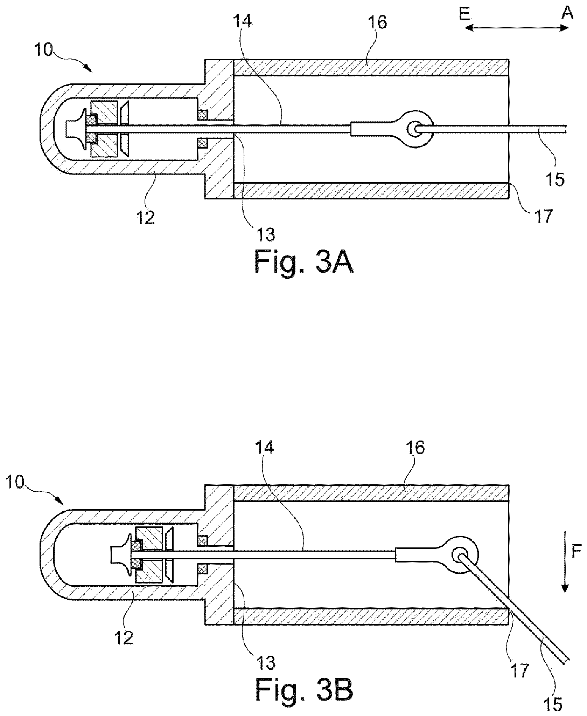

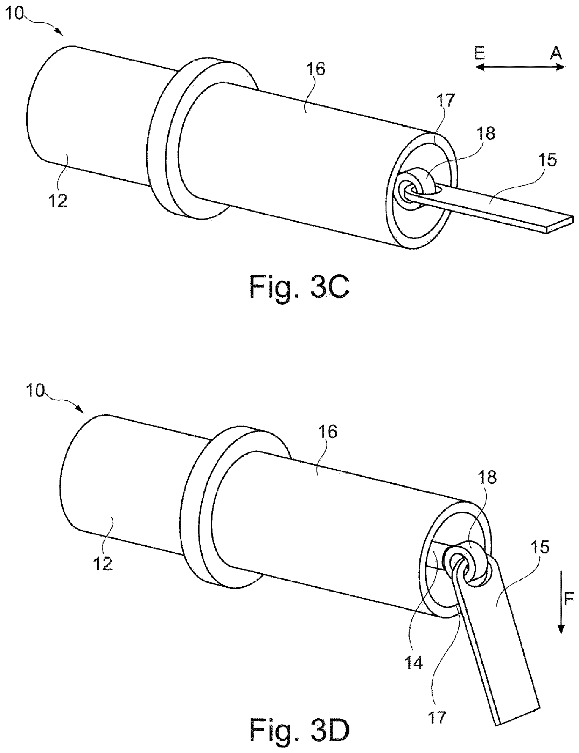

Figuren 3A und 3C zeigen eine dritte Ausführungsform der Vorrichtung 10 in einer Ausgangsposition. Die Ausgangsposition der Vorrichtung 10 ist in Figur 2A als Schnittdarstellung und in Figur 2C als perspektivische Darstellung gezeigt.

Die dritte Ausführungsform unterscheidet sich von den ersten und zweiten Ausführungsformen dadurch, dass der Vorsprung 16 als eine Hülse ausgeführt ist, welche einen umlaufenden Kontaktbereich 17 zum Kontaktieren des Kraftübertragungselements 15 aufweist. Der Kontaktbereich 17 ist dabei ringförmig an der Innenkante des Vorsprungs 16 ausgebildet.

Figuren 3B und 3D zeigen die dritte Ausführungsform der Vorrichtung 10 in einer Position, in der das Kraftübertragungselement 15 an dem Kontaktbereich 17 anliegt. Figur 3B zeigt diese Position anhand einer Schnittdarstellung und Figur 3D zeigt diese Position anhand einer perspektivischen Darstellung.

Führt die Kraft F zu einer weiteren Auslenkung des Kraftübertragungselements 15, zieht dieses das Interaktionselement 14 über das Anbindungselement 18 aus der Aufnahme 12 heraus, wobei das Herausziehen in Auszugsrichtung A über den Kontaktbereich 17 gehebelt erfolgt. In den Darstellungen der Figuren 3B und 3D liegt das Kraftübertragungselement 15 an einem unteren Bereich des Kontaktbereichs 17 an. Mithin zeigen die Figuren 3D und 3B das Interaktionselement 14 in einer Position, die gegenüber der in den Figuren 3A und 3C gezeigten Position einer herausgezogenen Position entspricht.

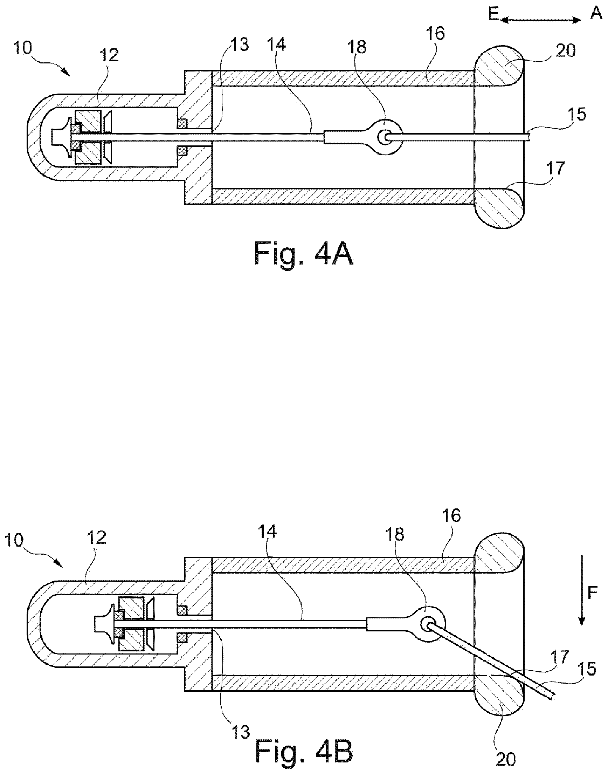

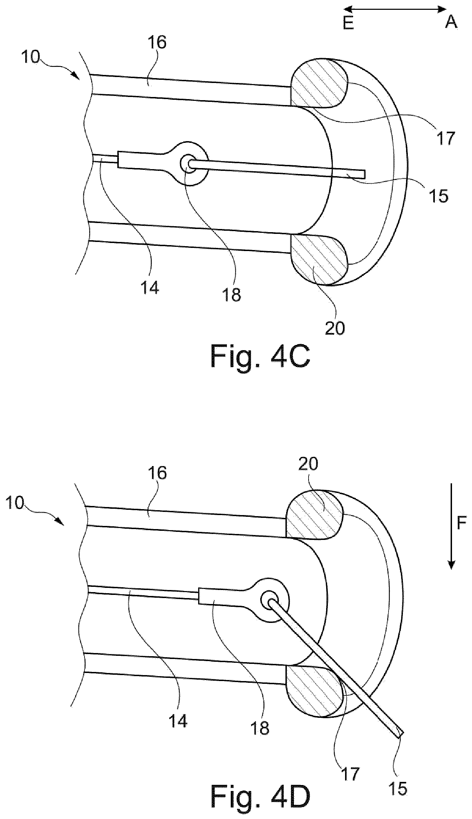

Figuren 4A und 4C zeigen eine vierte Ausführungsform der Vorrichtung 10 in einer Ausgangsposition. Die Ausgangsposition der Vorrichtung 10 ist in Figur 4A als Schnittdarstellung und in Figur 4C als perspektivische Darstellung einer Teilansicht der Vorrichtung 10gezeigt.

In dieser Ausführungsform ist am Vorsprung 16 ein Schwenkkopf 20 ausgebildet. In den übrigen Merkmalen ist die in Figur 4 offenbarte Vorrichtung identisch zu der Vorrichtung 10 gemäß der in den Figuren 3A-3D gezeigten Ausführungsform.

Figuren 4B und 4D zeigen die vierte Ausführungsform der Vorrichtung 10 in einer Position, in der das Kraftübertragungselement 15 an dem Kontaktbereich 17 anliegt. Figur 4B zeigt diese Position anhand einer Schnittdarstellung und Figur 4D zeigt diese Position anhand einer perspektivischen Darstellung einer Teilansicht der Vorrichtung 10.

In den in Figuren 4B und 4D abgebildeten Darstellungen bildet das Kraftübertragungselement 15 aufgrund einer anliegenden Kraft F einen Kontakt mit dem Kontaktbereich 17 aus. Der Kontaktbereich 17 ist dabei durch den ersten Schwenkkopf 20 ausgebildet. In den Darstellungen der Figuren 4B und 4D liegt das Kraftübertragungselement 15 an einem unteren Bereich des durch den ersten Schwenkkopf 20 ausgebildeten Kontaktbereichs 17 an. Mithin zeigen die Figuren 4D und 4B das Interaktionselement 14 in einer Position, die gegenüber der in den Figuren 4A und 4C gezeigten Position einer herausgezogenen Position entspricht.

Die Verkippungen, die für ein Kontaktieren des Kraftübertragungselements 15 mit dem Kontaktbereich 17 notwendig sind, sich aufgrund des Schwenkkopfes 20 deutlich reduziert. Für die praktische Anwendung der Vorrichtung 10 führt dies dazu, dass die Vorrichtung 10 auch bei kleineren Verkippungen sensibel reagieren kann, was sich positiv auf den erforderlichen Bewegungsspielraum der Vorrichtung 10 auswirkt.

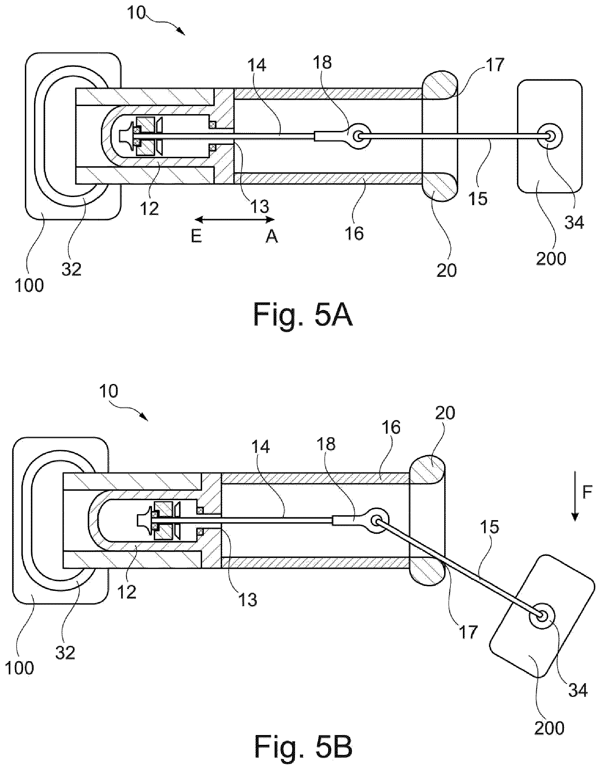

Figuren 5A und 5B zeigen eine fünfte Ausführungsform einer Vorrichtung 10 in einer Ausgangsposition und einer Position, in der das Kraftübertragungselement am Kontaktbereich anliegt, jeweils als Schnittzeichnung.

Aus den Figuren 5A und 5B ist ersichtlich, dass der Kontaktbereich 17 entlang der Oberfläche des ersten Schwenkkopfs 20 verläuft, wenn das Interaktionselement 14 aufgrund der Verkippung des

Kraftübertragungselements 15 weiter aus der Aufnahme 12 über die Aufnahmeöffnung 13 ausgefahren wird. In der Darstellung der Figur 5B liegt das Kraftübertragungselement 15 an einem unteren Bereich des durch den ersten Schwenkkopf 20 ausgebildeten Kontaktbereichs 17 an. Mithin zeigt die Figur 5B das Interaktionselement 14 in einer Position, die gegenüber der in der Figur 5A gezeigten Position einer herausgezogenen Position entspricht.

Darüber hinaus zeigen die Figuren 5A und 5B eine Vorrichtung 10 in einer Einbausituation mit weiteren Teilen zur Fixierung an einem menschlichen Körperbereich und/oder eines Sportgeräts. Dabei ist die Aufnahme 12 an einem ersten Teil 100 eines menschlichen Körperbereichs und/oder eines Sportgeräts fixiert und das Interaktionselement 14 über das Kraftübertragungselement 15 mit einem zweiten Teil 200 desselben menschlichen Körperbereichs und/oder desselben Sportgeräts fixiert. Wird der erste Teil 100 eines menschlichen Körperbereichs und/oder eines Sportgeräts relativ zum zweiten Teil 200 desselben menschlichen Körperbereichs und/oder desselben Sportgeräts bewegt ohne dass ein Kontakt zwischen dem Kontaktbereich 17 und dem Kraftübertragungselement 15 auftritt, ist die Dämpfung der Bewegung zwischen dem ersten Teil 100 und zweiten Teil 200 eines menschlichen Körperbereichs und/oder eines Sportgeräts proportional zum Hub, d. h. die Auslenkung des Interaktionselements 14 aus der Aufnahme 12 heraus bzw. in die Aufnahme 12 hinein.

Die Vorrichtung 10 ist mit dem ersten Teil 100 eines menschlichen Körperbereichs und/oder eines Sportgeräts über eine erste Befestigungsöse 32 und mit dem zweiten Teil 200 eines menschlichen Körperbereichs und/oder eines Sportgeräts über eine zweite Befestigungsöse 34 gelenkig verbunden. Dadurch kann die Schwenkbewegung bei Flexion/Extension möglichst reibungs- bzw. kraftarm ausgeführt werden. Alternativ können die erste Befestigungsöse 32 und/oder die zweite Befestigungsöse 34 auch als eine andere gelenkige Anbindung ausgeführt sein, insbesondere als ein Drehgelenk oder Kugelgelenk. Dadurch kann ein (Dreh-)Gelenk bereitgestellt werden, welches Gelenksbewegungen ermöglicht. Dies kann etwa bei Flexion / Extension des Sprunggelenks, oder aber auch am Kniegelenk vorteilhaft sein.

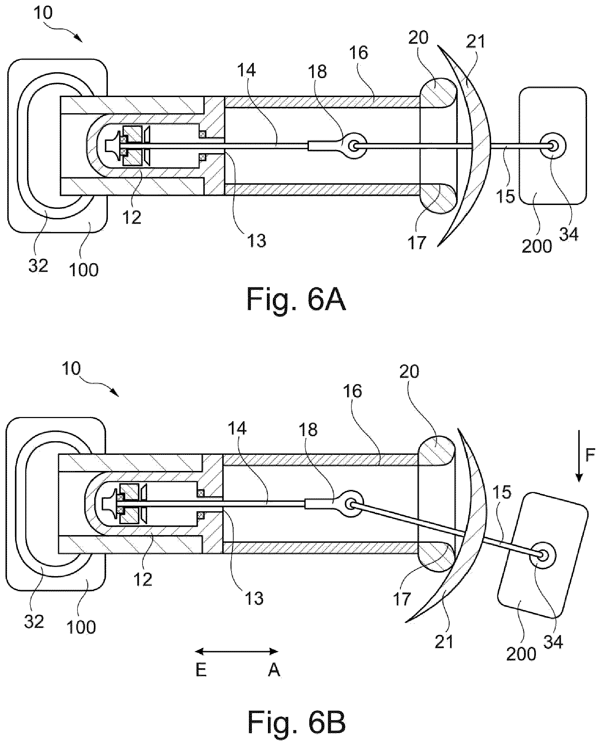

Figuren 6A und 6B zeigen eine sechste Ausführungsform einer Vorrichtung 10 in einer Ausgangsposition und in einer Position, in der ein Endanschlag 21 an einem Schwenkkopf 20 anliegt, jeweils als Schnittzeichnung. Auch hier ist die Vorrichtung 10 in einer Einbausituation abgebildet.

Die in den Figuren 6A und 6B offenbarte Vorrichtung 10 gemäß der sechsten Ausführungsform ist dahingehend modifiziert, dass diese einen Endanschlag 21 zum Kontaktieren des Schwenkkopfs 20 umfasst, um den Verschiebeweg des Kraftübertragungselements 15 in eine Einzugsrichtung E zu begrenzen. Der Endanschlag 21 ist starr am Kraftübertragungselement 15 befestigt und weist im gezeigten Ausführungsbeispiel einen halbmondförmigen Querschnitt auf, der zur Aufnahmeseite hin eine konkave Oberfläche aufweist. Der Kontaktbereich 17 des Schwenkkopfs 20 für die Kontaktierung des Kraftübertragungselements 15 ist in den in den Figuren 6A und 6B abgebildeten Darstellungen ein leicht versetzt in Bezug zu dem Kontaktpunkt des Endanschlags 21 mit dem Schwenckopf 20. Alternativ können der Kontaktbereich 17 und der Kontaktpunkt des Endanschlags 21 auch identische Bereiche sein.

An der zur Aufnahmeseite hin orientierten Innenseite weist der Endanschlag 21 gleitfördernde Eigenschaften auf, damit bei einem Anschlag desselben an dem Kontaktbereich 17 Bewegungsabläufe begünstigt werden können. In der Darstellung der Figur 5A liegt der Endanschlag 21 nicht am Schwenkkopf 20 an. In der Darstellung der Figur 5B liegt der Endanschlag 21 an einem unteren Bereich des Schwenkkopfs 20 an. Mithin zeigt die Figur 6B die Vorrichtung 10 in einem gestauchten Zustand. Das in Figur 6B abgebildete Interaktionselement 14 befindet sich relativ zur Darstellung der Figur 6A in einer Position, die einer gestauchten, jedoch nicht gehebelten Position der Vorrichtung 10 entspricht. Alternativ kann auch eine gestauchte und gehebelte Position der Vorrichtung 10 vorliegen.

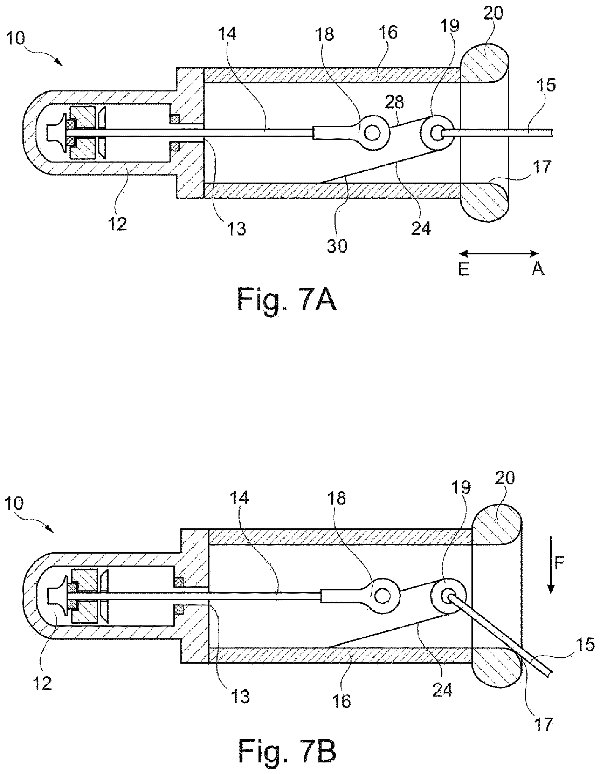

Die Figuren 7A und 7b zeigen eine siebte Ausführungsform einer Vorrichtung 10 in einer Ausgangsposition und einer Position, in der das Kraftübertragungselement 15 an einem Kontaktbereich 17 anliegt, jeweils als Schnittzeichnung.

Gemäß der siebten Ausführungsform umfasst die Vorrichtung 10 ein Umlenkelement 19 zum Umlenken einer auf das Kraftübertragungselement 15 wirkenden Kraft und ein Verbindungselement 24, wobei das Umlenkelement 19 an dem Kraftübertragungselement 15 angeordnet ist. Das Interaktionselement 14 ist dabei mit einem Ende 28 des Verbindungselements 24 verbunden. Der Vorsprung 16 ist mit dem anderen Ende 30 des Verbindungselements 24 verbunden. Ferner ist das Verbindungselement 24 über das Umlenkelement 19 am Interaktionselement 14 geführt.

In der in den Figuren 7A und 7B abgebildeten Darstellungen ist das Umlenkelement 19 eine Umlenkrolle und das Verbindungselement 24 ein Zugseil. Das Anbindungselement 18 ist als eine Öse ausgeführt. Die Umlenkrolle weist eine Laufrille zum Aufnehmen und Führen des Zugseil auf. Darüber hinaus weist die Umlenkrolle ein Lager zum rotierbaren Lagern der Umlenkrolle auf. Das Lager kann beispielsweise in Form eines Kugellagers ausgeführt sein. Die Umlenkrolle ist dabei am Kraftübertragungselement 15 angeordnet und steht über das Zugseil mit dem Interaktionselement 14 in Verbindung.

Erfolgt nur eine Verkippung des Kraftübertragungselements 15 aufgrund einer von außen anliegenden Kraft F, bildet das Kraftübertragungselement 15 in Abhängigkeit der Richtung der anliegenden Kraft F einen Kontakt mit dem Kontaktbereich 17 aus und gleitet unter Ausbildung eines Hebels in Auszugsrichtung A. Diese Bewegung wird über die Umlenkrolle sowie das Zugseil in eine Bewegung des Interaktionselements 14 übersetzt, wodurch eine Hubverdopplung erzielt wird. Das bedeutet, dass eine Bewegung des Kraftübertragungselements 15 in Auszugs- und Einzugsrichtung A bzw. E eine doppelt so große Bewegung des Kraftübertragungselements 14 in die entsprechende Auszugs- und Einzugsrichtung A bzw. E bewirkt. Die Umlenkelemente 19 sind aus Polypropylen (PP) gefertigt. Darüber hinaus kann die Umlenkrolle auch aus Metallen wie zum Beispiel Aluminium, Magnesium, Stahl oder anderen reibungsarmen Materialien gefertigt sein.

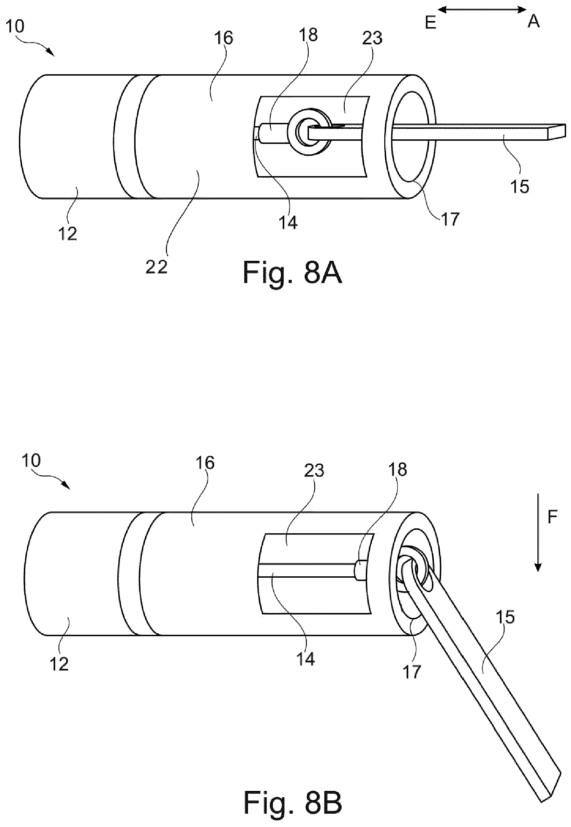

Die Figuren 8A und 8B zeigen eine achte Ausführungsform einer Vorrichtung in einer Ausgangsposition und einer Position, in der ein Kraftübertragungselement an einem Kontaktbereich anliegt, jeweils als Schnittzeichnung.

Gemäß der achten Ausführungsform umfasst der Vorsprung 16 eine Einhausung 22, die als eine Hülse ausgeführt ist. Zusätzlich ist innerhalb der Einhausung 22 ein Sichtfenster 23 ausgeführt, über das Teile des Interaktionselements 14, das Anbindungselement 18 sowie des Kraftübertragungselements 15 visuell betrachtet werden können.

In der Darstellung der Figur 8B liegt das Kraftübertragungselement 15 an einem unteren Bereich des Kontaktbereichs 17 an. Mithin zeigt die Figur 8B das Interaktionselement 14 in einer Position, die gegenüber der in Figur 8A gezeigten Position einer herausgezogenen Position entspricht.

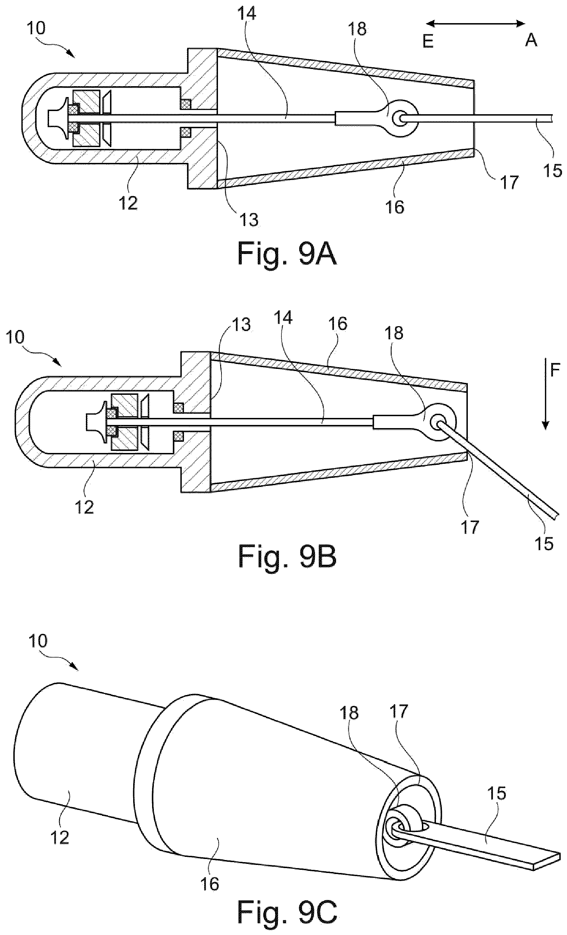

Figuren 9A und 9B zeigen eine neunte Ausführungsform einer Vorrichtung in einer Ausgangsposition und einer Position, in der ein Kraftübertragungselement an einem Kontaktbereich anliegt, als Schnittzeichnung. Figur 9C veranschaulicht die neunte Ausführungsform der Vorrichtung 10 in der in Figur 9A abgebildeten Position anhand einer perspektivischen Darstellung.

Gemäß der neunten Ausführungsform erstreckt sich der Vorsprung 16 derart konisch in die Auszugsrichtung A des Interaktionselements 14, dass der Abstand zwischen einer Längsachse des Interaktionselements 14 und dem Vorsprung 16 in Auszugsrichtung A abnimmt. Der Vorsprung 16 weist dabei einen umlaufenden Kontaktbereich 17 auf. Mithin zeigt die Figur 9B das Interaktionselement 14 in einer Position, die gegenüber der in den Figuren 9A und 9C gezeigten Position einer herausgezogenen Position entspricht.

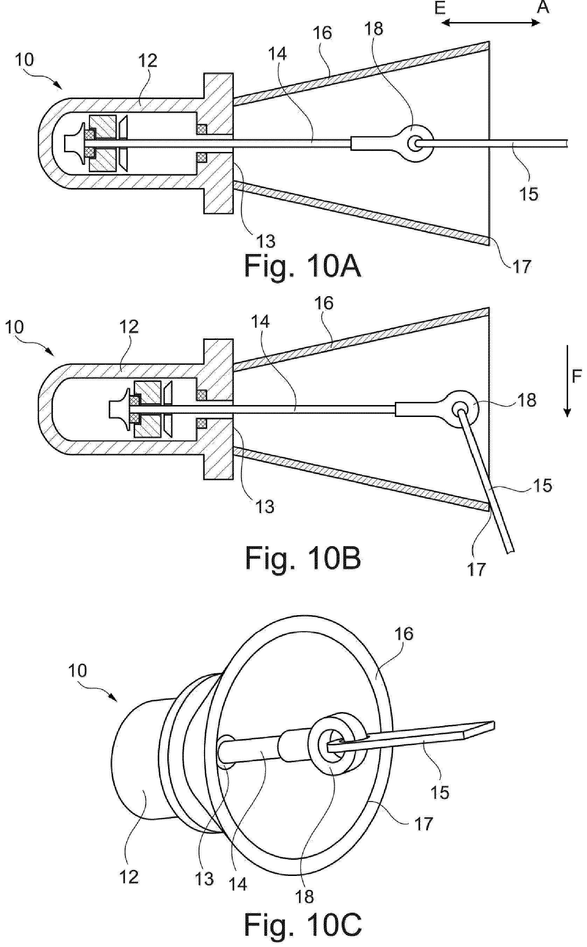

Figuren 10A und 10B zeigen eine zehnte Ausführungsform einer Vorrichtung in einer Ausgangsposition und einer Position, in der ein Kraftübertragungselement an einem Kontaktbereich anliegt, als Schnittzeichnung. Figur 10C veranschaulicht die zehnte Ausführungsform der Vorrichtung 10 in der in Figur 10A abgebildeten Position anhand einer perspektivischen Darstellung.

Gemäß der zehnten Ausführungsform erstreckt sich der Vorsprung 16 derart konisch in die Auszugsrichtung A des Interaktionselements 14, dass der Abstand zwischen einer Längsachse des Interaktionselements 14 und dem Vorsprung 16 in Auszugsrichtung A zunimmt. Der Vorsprung 16 weist dabei einen umlaufenden Kontaktbereich 17 auf. Mithin zeigt die Figur 10B das Interaktionselement 14 in einer Position, die gegenüber der in den Figuren 10A und 10C gezeigten Position einer herausgezogenen Position entspricht.

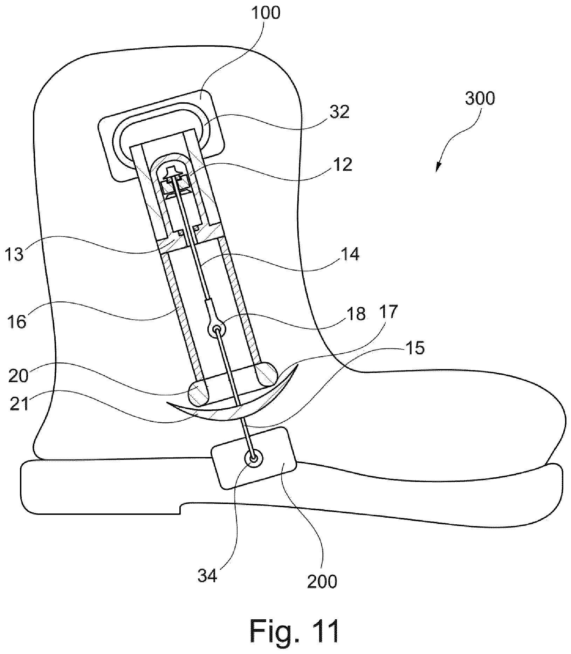

Figur 11 zeigt schematisch die Seitenansicht eines nicht erfindungsgemäßen Schuhs 300 mit einer Vorrichtung 10 gemäß den Figuren 6A und 6B. Die Vorrichtung 10 dient dabei zur Stabilisierung von Bewegungen zwischen dem Schien- bzw. Wadenbein und dem Sprungbein, um etwa einer Verstauchung des Sprunggelenks (Distorsion) vorzubeugen. Genauer gesagt schützt die Vorrichtung 10 im gezeigten Fall vor einem Supinationstrauma, das durch Abknicken über den Außenknöchel hinweg entsteht und zumeist mit einer Schädigung der Außenbänder einhergeht. In dem in Figur 11 abgebildeten Schuh 300 ist die fixierbare Aufnahme 12 an einem ersten Teil 100 eines Schuhs 300 befestigt, der dem Bewegungsablauf des Wadenbeins folgt. Das fixierbare Interaktionselement 14 ist mithin an einem zweiten Teil 200 desselben Schuhs 300 befestigt, der dem Bewegungsablauf des Sprungbeins folgt. In der gezeigten Ausführungsform ist der erste Teil 100 des Schuhs 300 Teil eines Schuhschafts 310 und der zweite Teil 200 des Schuhs 300 Teil eines Schuhboden 320.

Die Stabilisierung erfolgt dabei nicht nur gegenüber unphysiologischen Kräften entlang der Auszugsrichtung A bzw. Einzugsrichtung E der Vorrichtung 10, sondern auch gegenüber Kräften, die winklig dazu auftreten, etwa 45 ° oder 90 ° in Abbildungsebene gekippt zur Auszugsrichtung A bzw. Einzugsrichtung E. Aufgrund des Kraftübertragungselements 15, das schwenkbar an dem aus der Aufnahme 12 ragenden Ende des Interaktionselements 14 gehalten ist, bewirken derart winklig verschränkte Kräfte, dass das Kraftübertragungselement 15 verkippt wird und an einem ersten Kontaktbereich 17 eines ersten Vorsprungs 16 mit diesem in Kontakt kommt. Dadurch erfolgt eine Hebelung des Kraftübertragungselements 15 am ersten Kontaktbereich 17, wodurch das Interaktionselement 14 aus der Aufnahme 12 herausgezogen wird.

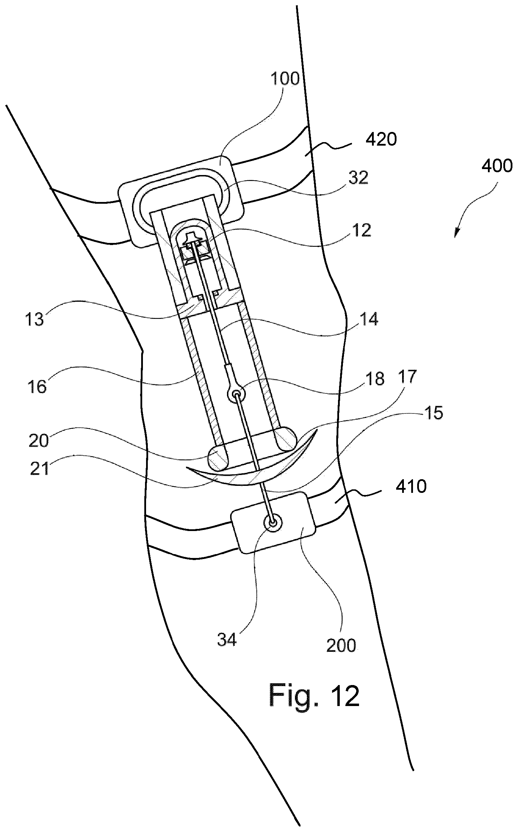

Figur 12 zeigt schematisch eine Seitenansicht einer nicht erfindungsgemäßen Kniemanschette 400 an einem menschlichen Knie mit einer Vorrichtung 10 gemäß der sechsten Ausführungsform in einer Ausgangsposition. Alternativ zu der Vorrichtung 10 gemäß der sechsten Ausführung kann auch eine Vorrichtung 10 gemäß einer der übrigen Ausführungsformen in die Kniemanschette 400 integriert sein. In der gezeigten Abbildung ist die Vorrichtung über eine erste Befestigungsöse 32 an einem ersten Teil 100 bzw. an einem oberen Befestigungsgurt 420, und über eine erste Befestigungsöse 34 an einem zweiten Teil 200 bzw. an einem unteren Befestigungsgurt 410 befestigt. Durch diese Befestigung erfolgt eine Relativbewegung der ersten und zweiten Teile 100 und 200 relativ zueinander, wenn das Knie gebeugt wird. Bei der Beugung des Knies kommt das Kraftübertragungselement 15 in einen Kontakt mit dem Kontaktbereich 17, wodurch das Interaktionselement 14 unter einer geschwindigkeitsabhängigen Hebelwirkung aus der Aufnahme 12 gefahren wird.

Bei einer langsamen Beugung des Knies bleibt die Vorrichtung 10 flexibel, das heißt, die Verdrängung des Füllmediums in der Aufnahme 12 durch das Interaktionselement 14 erfolgt reibungsarm. Bei einer schnellen Beugung des Knies hingegen bewirkt die Verdrängung des Füllmediums in der Aufnahme 12 durch das Interaktionselement 14 zu einem erhöhten Widerstand, wodurch die Vorrichtung versteift und eine stützende Wirkung auf das Kniegelenk aufweist.

Aus illustrativen Zwecken ist die Vorrichtung 10 in Figur 12 vergrößert dargestellt. Dadurch entfaltet die Kniemanschette 400 eine geschwindigkeitsabhängige Stützwirkung. Gegenüber herkömmlichen Stabilisierungsschienen hat dies den Vorteil, dass sich der Träger der Kniemanschette 400 motorisch frei bewegen kann, solange die Bewegungsgeschwindigkeit eine Grenzgeschwindigkeit nicht erreicht und erst dann eine stützende Wirkung der Kniemanschette 400 wahrnimmt, wenn die Bewegungsgeschwindigkeit die Grenzgeschwindigkeit erreicht oder überschreitet.

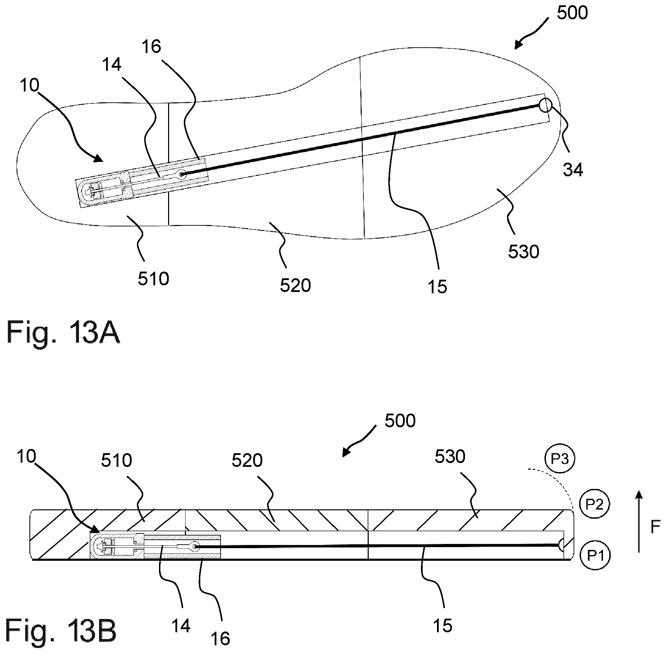

Figur 13A zeigt eine schematische Draufsicht von unten auf den Schnitt durch eine Schuhsohle 500 mit einer Vorrichtung 10 gemäß der dritten Ausführungsform in einer Ausgangsposition. Die Schuhsohle 500 ist aufgeteilt in einen steifen Bereich 510, einen ersten flexiblen Bereich Flex-Zone 520 und einen zweiten flexiblen Bereich Flex-Zone 530. Die Aufnahme 12 ist im steifen Bereich 510 fixiert und ragt mit dem Vorsprung 16 bis in den ersten flexiblen Bereich Flex-Zone 520 hinein. Der erste flexible Bereich Flex-Zone 1 ist flexibler als der zweite flexible Bereich Flex-Zone 2. Das Kraftübertragungselement 15 ist über eine zweite Befestigungsöse 34 am distalen Ende des zweiten flexiblen Bereichs 530 befestigt. Alternativ kann die Schuhsole auch über ihren gesamten Bereich eine einheitliche Flexibilität aufweisen.

Figur 13B zeigt schematisch eine Querschnittszeichnung der Schuhsohle 500 aus Figur 13A. Greift eine Kraft F an der Schuhsohle 500 an, gelangt die Schuhsohle aus einer Ausgangsposition P1 in eine zweite Position P2 und letztlich in eine dritte Position P3. Nach Verlassen der Position P1 wird ein Kontakt des Kraftübertragungselements 15 mit dem Vorsprung 16 ausgebildet, wodurch das Interaktionselement 14 unter einer Hebelwirkung aus der Aufnahme gefahren wird.

Die Steifigkeit der Schuhsohle 500 ist abhängig von der Geschwindigkeit, mit der die Schuhsohle 500 gekrümmt wird. Bei einer langsamen Krümmung, wie sie etwa beim Gehen auftritt, wird zwar das Interaktionselement 14 über das Kraftübertragungselement 15 gehebelt aus der Aufnahme 12 heraus- und in die Aufnahme 12 eingefahren, allerdings erfolgt die Verdrängung des Füllmediums durch das Interaktionselement 14 derart langsam, dass die Vorrichtung nicht blockiert. In diesem Zustand ist die Sohle flexibel. Bei einer schnellen Krümmung, wie sie etwa beim Sprinten auftritt, ruft die Verdrängung des Füllmediums durch das Interaktionselement 14 einen erhöhten Widerstand hervor, wodurch die Schuhsohle 500 versteift. Durch die integrierte Vorrichtung 10 kann die Schuhsohle 500 eine geschwindigkeitsabhängige Stützwirkung entfalten.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezugszeichenliste

- 10: Vorrichtung
- 12: Aufnahme
- 13: Aufnahmeöffnung
- 14: Interaktionselement
- 15: Kraftübertragungselement
- 16: Erster Vorsprung
- 16': Zweiter Vorsprung
- 17: Erster Kontaktbereich
- 17': Zweiter Kontaktbereich
- 18: Anbindungselement
- 19: Umlenkelement

- 20: Schwenkkopf
- 21: Endanschlag
- 22: Einhausung
- 23: Sichtfenster
- 24: Verbindungselement
- 26: orthogonale Ebene
- 28: Erstes Ende des Verbindungselements

- 30: Zweites Ende des Verbindungselements
- 32: Erste Befestigungsöse
- 34: Zweite Befestigungsöse

- 100: Erster Teil eines menschlichen Körperbereichs und/oder eines Sportgeräts

- 200: Zweiter Teil desselben menschlichen Körperbereichs und/oder desselben Sportgeräts

- 300: Schuh
- 310: Schuhschaft
- 320: Schuhboden

- 400: Kniemanschette
- 410: Unterer Befestigungsgurt
- 420: Oberer Befestigungsgurt

- 500: Schuhsohle
- 510: Steifer Bereich
- 520: Flex-Zone 1
- 530: Flex-Zone 2

- A: Auszugsrichtung
- E: Einzugsrichtung
- F: Kraft

- P1: Erste Position
- P2: Zweite Position
- P3: Dritte Position

## Patentansprüche

1. Schuhsohle (500) umfassend eine Vorrichtung (10) zur Stabilisierung von Bewegungen zweier sich relativ zueinander bewegbarer Teile der Schuhsohle, umfassend:
eine an einem ersten Teil der Schuhsohle fixierbare Aufnahme (12), wobei die Aufnahme (12) mit einem Füllmedium gefüllt ist,
wobei die Aufnahme (12) eine Aufnahmeöffnung (13) umfasst,
wobei die Vorrichtung (10) ferner ein Kraftübertragungselement (15) umfasst, und
ein an einem zweiten Teil der Schuhsohle (500) über das Kraftübertragungselement (15) fixierbares Interaktionselement (14), das zumindest teilweise in der Aufnahme (12) verschiebbar aufgenommen ist und sich durch die Aufnahmeöffnung (13) erstreckt,
wobei der Teil des Interaktionselements (14), der sich in der Aufnahme (12) befindet, mit dem Füllmedium in Kontakt steht,
**dadurch gekennzeichnet, dass**
das Kraftübertragungselement (15) schwenkbar an dem aus der Aufnahme (12) ragenden Ende des Interaktionselements (14) gehalten ist,
wobei sich von der Aufnahme (12) im Bereich der Aufnahmeöffnung (13) mindestens ein Vorsprung (16) in eine Auszugsrichtung (A) des Interaktionselements (14), entlang der das Interaktionselement (14) in der Aufnahme verschoben werden kann, erstreckt, wobei der Vorsprung (16) mindestens einen ersten Kontaktbereich (17) zum Kontaktieren des Kraftübertragungselements (15) umfasst.

2. Schuhsohle (500) gemäß Anspruch 1, **dadurch gekennzeichnet, dass** der Vorsprung (16) mindestens einen zweiten Kontaktbereich (17`) zum Kontaktieren des Kraftübertragungselements (15) umfasst, wobei der mindestens eine zweite Kontaktbereich (17`) dem mindestens einen ersten Kontaktbereich (17) in einer zur Auszugsrichtung (A) orthogonalen Ebene (26) und in Bezug zur Auszugsrichtung (A) gegenüberliegt.

3. Schuhsohle (500) gemäß Anspruch 1 oder 2, **dadurch gekennzeichnet, dass** die Vorrichtung (10) weiter ein erstes Anbindungselement (18) zum Verbinden des Interaktionselements (14) mit dem Kraftübertragungselement (15) umfasst, wobei das Anbindungselement (18) eine Öse oder ein Gelenk, ein Drehgelenk oder ein Kugelgelenk, umfasst, wobei das Anbindungselement (18) integral mit dem Interaktionselement (14) oder dem Kraftübertragungselement (15) ausgebildet ist.

4. Schuhsohle (500) gemäß einem der vorhergehenden Ansprüche, **dadurch gekennzeichnet, dass** zumindest der erste Kontaktbereich (17) mindestens einen Schwenkkopf (20) umfasst, wobei das Kraftübertragungselement (15) den mindestens einen Schwenkkopf (20) kontaktieren und um diesen schwenken kann, wobei zumindest ein Teil des mindestens einen Schwenkkopfs (20) einen kreisförmigen oder elliptischen Abschnitt zum Kontaktieren des Kraftübertragungselements (15) umfasst.

5. Schuhsohle (500) gemäß einem der vorhergehenden Ansprüche, **dadurch gekennzeichnet, dass** die Vorrichtung (10) ferner ein zweites Anbindungselement (18) zum Verbinden des Kraftübertragungselements (15) mit dem zweiten Teil des menschlichen Körperbereichs und/oder des Sportgeräts umfasst, wobei das zweite Anbindungselement (18) eine Öse oder ein Gelenk, ein Drehgelenk oder ein Kugelgelenk umfasst.

6. Schuhsohle (500) gemäß einem der vorhergehenden Ansprüche, **dadurch gekennzeichnet, dass** das Kraftübertragungselement (15) einen Endanschlag (21) zum Kontaktieren des mindestens einen ersten Kontaktbereichs (17) umfasst, um einen Verschiebeweg des Kraftübertragungselements (15) in eine Einzugsrichtung (E) zu begrenzen.

7. Schuhsohle (500) gemäß einem der vorhergehenden Ansprüche, **dadurch gekennzeichnet, dass** der Vorsprung (16) eine Einhausung (22) umfasst, wobei die Einhausung (22) zumindest einen Teil des sich aus der Aufnahmeöffnung (13) der Aufnahme (12) erstreckenden Interaktionselements (14) umgibt.

8. Schuhsohle (500) gemäß Anspruch 7, **dadurch gekennzeichnet, dass** die Einhausung (22) ein Sichtfenster (23) umfasst.

9. Schuhsohle (500) gemäß einem der vorhergehenden Ansprüche, **dadurch gekennzeichnet, dass** sich der Vorsprung (16) konisch in die Auszugsrichtung (A) des Interaktionselements (14) erstreckt, derart, dass der Abstand zwischen einer Längsachse (L) des Interaktionselements (14) und dem Vorsprung (16) in Auszugsrichtung (A) abnimmt oder zunimmt.

10. Schuhsohle (500) gemäß einem der vorhergehenden Ansprüche, **dadurch gekennzeichnet, dass** die Vorrichtung (10) ferner ein Umlenkelement (19) zum Umlenken einer auf das Kraftübertragungselement (15) wirkenden Kraft und ein Verbindungselement (24) umfasst, wobei das Umlenkelement (19) an dem Kraftübertragungselement (15) angeordnet ist, wobei das Interaktionselement (14) mit dem einen Ende des Verbindungselements (24) und der mindestens eine Vorsprung (16) oder die Aufnahme (12) mit dem anderen Ende des Verbindungselements (24) verbunden sind, und wobei das Verbindungselement (24) über das Umlenkelement (19) am Interaktionselement (14) geführt ist.

11. Schuhsole (500) gemäß einem der vorhergehenden Ansprüche, wobei die Schuhsohle mindestens zwei Bereiche (510, 520, 530) unterschiedlicher Flexibilität aufweist.

12. Schuh (300) umfassend eine Schuhsole (500) gemäß einem der vorhergehenden Ansprüche.

## Claims

1. A shoe sole (500) comprising a device (10) for stabilising movements of two parts of the shoe sole (500) that are movable relative to each other, comprising:
a receptacle (12), which is fixable to a first part of the shoe sole (500), wherein the receptacle (12) is filled with a filling medium, and wherein the receptacle (12) comprises a receiving opening (13),
wherein the device (10) further comprises a force transmission element (15), and
an interaction element (14) fixable on a second part of the shoe sole (500) via the force transmission element (15), which is at least partially accommodated displaceably in the receptacle (12), and extends through the receiving opening (13), wherein the part of the interaction element (14) that is located in the receptacle (12) is in contact with the filling medium,
**characterized in that**
the force transmission element (15) is held pivotably on the end of the interaction element (14) which protrudes out of the receptacle (12),
wherein at least one projection (16) extends from the receptacle (12), in the region of the receiving opening (13), in an outward direction (A) of the interaction element (14) along which the interaction element (14) can be displaced in the receptacle, wherein the projection (16) comprises at least one first contact region (17) for contacting the force transmission element (15).

2. A shoe sole (500) according to claim 1, **characterized in that** the projection (16) comprises at least one second contact region (17') for contacting the force transmission element (15), wherein the at least one second contact region (17') is located opposite the at least one first contact region (17) in a plane (26) orthogonal to the outward direction (A) and in relation to the outward direction (A).

3. A shoe sole (500) according to claim 1 or 2, **characterized in that** the device (10) further comprises a first attachment element (18) for connecting the interaction element (14) with the force transmission element (15), wherein the attachment element (18) comprises an eyelet or a joint, a rotary joint or a ball joint, wherein the attachment element (18) is formed integrally with the interaction element (14) or the force transmission element (15).

4. A shoe sole (500) according to any of the preceding claims, **characterized in that** at least the first contact region (17) comprises at least one pivot head (20), wherein the force transmission element (15) can contact about the at least at least one pivot head (20) and pivot about it, wherein at least a part of the at least one pivot head (20) comprises a circular or elliptical section for contacting the force transmission element (15).

5. A shoe sole (500) according to any of the preceding claims, **characterized in that** the device (10) further comprises a second attachment element (18) for connecting the force transfer element (15) with the second part of the human body region and/or the sports appliance, wherein the second attachment element (18) comprises an eyelet or a joint, a rotary joint or a ball joint.

6. A shoe sole (500) according to one of the preceding claims, **characterized in that** the force transmission element (15) comprises an end stop (21) for contacting the at least one first contact region (17), in order to limit a displacement path of the force transmission element (15) in a inward direction (E).

7. A shoe sole (500) according to one of the preceding claims, **characterized in that** the projection (16) comprises a housing (22), wherein the housing (22) surrounds at least a part of the interaction element (14) extending out of the receiving opening (13) of the receptacle (12).

8. A shoe sole (500) according to claim 7, **characterized in that** the housing (22) comprises a viewing window (23).

9. A shoe sole (500) according to one of the preceding claims, **characterized in that** the projection (16) extends conically in the outward direction (A) of the interaction element (14), in such a way that the distance between a longitudinal axis (L) of the interaction element (14) and the projection (16) decreases or increases in the outward direction (A).

10. A shoe sole (500) according to one of the preceding claims, **characterized in that** the device (10) further comprises a deflection element (19), for deflecting a force acting on the force transmission element (15) and a connection element (24), wherein the deflection element (19) is arranged on the force transmission element (15), wherein the interaction element (14) is connected to the one end of the connection element (24) and the at least one projection (16) or the receptacle (12) is connected to the other end of the connection element (24), and wherein the connection element (24) is guided by the deflection element (19) on the interaction element (14).

11. A shoe sole (500) according to one of the preceding claims, wherein the shoe sole (500) has at least two regions (510, 520, 530) of different flexibility.

12. A shoe (300) comprising a shoe sole (500) according to one of the preceding claims.

## Revendications

1. Semelle de chaussure (500) comprenant un dispositif (10) pour stabiliser des mouvements de deux parties de la semelle de chaussure déplaçables l'une par rapport à l'autre, comprenant :
un logement (12) pouvant être fixé à une première partie de la semelle de chaussure, dans laquelle le logement (12) est rempli d'un milieu de remplissage,
dans laquelle le logement (12) comprend une ouverture de logement (13),
dans laquelle le dispositif (10) comprend en outre un élément de transmission de force (15), et
un élément d'interaction (14) pouvant être fixé à une seconde partie de la semelle de chaussure (500) par l'intermédiaire de l'élément de transmission de force (15), élément qui est logé au moins partiellement de manière déplaçable dans le logement (12) et s'étend à travers l'ouverture de logement (13),
dans laquelle la partie de l'élément d'interaction (14) qui se trouve dans le logement (12) est en contact avec le milieu de remplissage,
**caractérisé en ce que**
l'élément de transmission de force (15) est maintenu de manière pivotante au niveau de l'extrémité de l'élément d'interaction (14) dépassant du logement (12),
dans laquelle au moins une saillie (16) s'étend du logement (12) dans la zone de l'ouverture de logement (13) dans une direction d'extraction (A) de l'élément d'interaction (14), le long de laquelle l'élément d'interaction (14) peut être déplacé dans le logement, dans laquelle la saillie (16) comprend au moins une première zone de contact (17) pour la mise en contact de l'élément de transmission de force (15).

2. Semelle de chaussure (500) selon la revendication 1, **caractérisée en ce que** la saillie (16) comprend au moins une seconde zone de contact (17') pour la mise en contact de l'élément de transmission de force (15), dans laquelle la au moins une seconde zone de contact (17') est opposée à la au moins une première zone de contact (17) dans un plan (26) orthogonal à la direction d'extraction (A) et par rapport à la direction d'extraction (A).

3. Semelle de chaussure (500) selon la revendication 1 ou 2, **caractérisée en ce que** le dispositif (10) comprend en outre un premier élément de liaison (18) pour connecter l'élément d'interaction (14) à l'élément de transmission de force (15), dans laquelle l'élément de liaison (18) comprend un anneau ou une articulation, un pivot ou un joint à rotule, dans laquelle l'élément de liaison (18) est conçu d'un seul tenant avec l'élément d'interaction (14) ou l'élément de transmission de force (15).

4. Semelle de chaussure (500) selon l'une quelconque des revendications précédentes, **caractérisée en ce qu'**au moins la première zone de contact (17) comprend au moins une tête pivotante (20), dans laquelle l'élément de transmission de force (15) est capable d'entrer en contact avec et de pivoter autour de la au moins une tête pivotante (20), dans laquelle au moins une partie de la au moins une tête pivotante (20) comprend une section circulaire ou elliptique pour la mise en contact de l'élément de transmission de force (15).

5. Semelle de chaussure (500) selon l'une quelconque des revendications précédentes, **caractérisée en ce que** le dispositif (10) comprend en outre un second élément de liaison (18) pour connecter l'élément de transmission de force (15) à la seconde partie de la zone de corps humain et/ou de l'équipement sportif, dans laquelle le second élément de liaison (18) comprend un anneau ou une articulation, un pivot ou un joint à rotule.

6. Semelle de chaussure (500) selon l'une quelconque des revendications précédentes, **caractérisée en ce que** l'élément de transmission de force (15) comprend une butée de fin de course (21) pour la mise en contact avec la au moins une première zone de contact (17) pour limiter une course de déplacement de l'élément de transmission de force (15) dans une direction d'introduction (E).

7. Semelle de chaussure (500) selon l'une quelconque des revendications précédentes, **caractérisée en ce que** la saillie (16) comprend une enceinte (22), dans laquelle l'enceinte (22) entoure au moins une partie de l'élément d'interaction (14) s'étendant hors de l'ouverture de logement (13) du logement (12).

8. Semelle de chaussure (500) selon la revendication 7, **caractérisée en ce que** l'enceinte (22) comprend une fenêtre de visualisation (23).

9. Semelle de chaussure (500) selon l'une quelconque des revendications précédentes, **caractérisée en ce que** la saillie (16) s'étend de manière conique dans la direction d'extraction (A) de l'élément d'interaction (14) de sorte que la distance entre un axe longitudinal (L) de l'élément d'interaction (14) et la saillie (16) diminue ou augmente dans la direction d'extraction (A).

10. Semelle de chaussure (500) selon l'une quelconque des revendications précédentes, **caractérisée en ce que** le dispositif (10) comprend en outre un élément de renvoi (19) pour renvoyer une force agissant sur l'élément de transmission de force (15) et un élément de connexion (24), dans laquelle l'élément de renvoi (19) est disposé au niveau de l'élément de transmission de force (15), dans laquelle l'élément d'interaction (14) est connecté à une extrémité de l'élément de connexion (24) et la au moins une saillie (16) ou le logement (12) sont connectés à l'autre extrémité de l'élément de connexion (24), et l'élément de connexion (24) est guidé sur l'élément d'interaction (14) par l'intermédiaire de l'élément de renvoi (19).

11. Semelle de chaussure (500) selon l'une quelconque des revendications précédentes, dans laquelle la semelle de chaussure présente au moins deux zones (510, 520, 530) de flexibilité différente.

12. Chaussure (300) comprenant une semelle de chaussure (500) selon l'une quelconque des revendications précédentes.
